(19) Europäisches Patentamt

European Patent Office

Office européen des brevets

(11) **EP 2 617 733 A1**

(12) **EUROPEAN PATENT APPLICATION**

(43) Date of publication:
**24.07.2013 Bulletin 2013/30**

(51) Int Cl.:
***C07K 14/79*** *(2006.01)*

(21) Application number: **13163795.1**

(22) Date of filing: **05.02.2010**

(84) Designated Contracting States:
**AT BE BG CH CY CZ DE DK EE ES FI FR GB GR
HR HU IE IS IT LI LT LU LV MC MK MT NL NO PL
PT RO SE SI SK SM TR**

(30) Priority: **06.02.2009 EP 09152332**

(62) Document number(s) of the earlier application(s) in
accordance with Art. 76 EPC:
**10705118.7 / 2 393 834**

(71) Applicant: **Novozymes Biopharma DK A/S
2880 Bagsvaerd (DK)**

(72) Inventors:
• **Blackwell, Lee
Nottingham
Nottinghamshire NG14 5HU (GB)**

• **Cameron, Jason
Nottingham
Nottinghamshire NG3 6HB (GB)**
• **Morton, Philip Harvey
Nottingham
Nottinghamshire NG2 6FS (GB)**

(74) Representative: **Hansen, Marianne Rudolph
Novozymes A/S
Patents
Krogshøjvej 36
2880 Bagsværd (DK)**

Remarks:
This application was filed on 15-04-2013 as a
divisional application to the application mentioned
under INID code 62.

(54) **Purification process**

(57) A process for purifying a transferrin solution and the product of the purification process. A process for producing transferrin and the product of the production process. Use of the transferrin in cell culture, as a pharma- ceutical ingredient, as a pharmaceutical product and in a cell culture medium.

EP 2 617 733 A1

**Description**

**Reference to sequence listing**

This application contains a Sequence Listing in computer readable form. The computer readable form is incorporated herein by reference.

## FIELD OF THE INVENTION

The invention relates to a process for purifying a transferrin solution. The invention also relates to the product of the purification process. The term 'transferrin' refers to both transferrin and transferrin-like proteins.

## BACKGROUND TO THE INVENTION

The listing or discussion of a prior-published document in this specification should not necessarily be taken as an acknowledgement that the document is part of the state of the art or is common general knowledge.
Transferrin is a protein that is known to regulate the bioavailability of iron to the cell, supporting key metabolic processes such as DNA synthesis and oxygen transport. Human serum transferrin (HST) is the major iron-binding protein in normal human plasma, and is present at about 2-4 g/l (van Campenhout et al, 2003, Free Radic. Res., 37, 1069-1077). Serum transferrin is currently derived from either bovine or human sources and is a common supplement used in commercial scale mammalian cell culture. With increasing pressure from regulatory authorities to reduce animal-derived components in the manufacture of biologics, recombinant transferrin is desirable for the serum-free, large scale mammalian cell culture industry.
More specifically, human plasma transferrin is a monomeric glycoprotein of 678 amino acids with a relative molecular mass of about 80 kDa. Each molecule of transferrin can bind strongly one or two ferric ions.
Purification of transferrin from plasma by chromatography has been described, for example in Rivat et al (1992) Journal of Chromatography, 576: 71-77, US 6,251,860; US 5,744,586; and US 5,041,537. Purification of recombinant transferrin from yeast has been described in PCT/EP2008/060482, and US 5,986,067. Transferrin may also be produced in other hosts, such as in plants
Irrespective of the source of transferrin, be it animal, human or recombinant, it is desirable to recover maximal amounts of transferrin during purification of the transferrin from the source. However, current methods of purification of transferrin do not result in a sufficiently high yield of purified transferrin. Therefore, what is required is an improved purification process for transferrin.

## SUMMARY OF THE INVENTION

The invention provides a chromatographic purification process which results in an increased yield of transferrin. Two steps of the purification process have been found to be particularly beneficial. These two steps may be used independently or together.
Accordingly, the invention provides an improved process for the purification of transferrin from a relatively impure solution of transferrin. The process may include one or both of an anionic chromatography step and a cationic chromatography step.
The invention also provides transferrin purified by the purification method and uses of the transferrin purified by the purification method.

## BRIEF DESCRIPTION OF DRAWINGS

Fig. 1 shows A254nm trace of Capto™Q chromatograph with (A) zero and (B) 824 mol.mole$^{-1}$ (5.4 mM) borate present in the transferrin solution which is loaded onto the chromatography column.
Fig. 2 is a graphical representation, overlay plot, of the SP-FF low pH, high salt wash 2 design space for a host cell protein clearance of at least 275-fold and a recovery of at least 60%.

## DETAILED DESCRIPTION OF THE INVENTION

A first aspect of the invention provides a process for purifying a transferrin solution (the transferrin solution may be considered to be a 'starting material'), the process comprising:

a) adding tetraborate to a relatively impure solution of transferrin to provide a solution of transferrin and tetraborate;

b) applying the solution of transferrin and tetraborate to an anionic chromatographic material for which the transferrin has no specific binding activity such that the transferrin binds to the material;

c) washing the chromatographic material to which the transferrin is bound;

d) optionally further washing the chromatographic material to which the transferrin is bound; and

e) optionally, recovering the transferrin from the anionic chromatographic material.

Preferably the wash of step (c) is carried out using a wash solution having a lower concentration than the wash solution used in the wash of step (d). For example, 'lower' may mean at least 5, 10, 25, 30 or 50% lower and/or by at least 5, 10, 20, 30, 40, or 50 mM or by at least 2-, 3-, 4-, 5- or 10-fold.

More specifically, the process for purifying a transferrin solution may comprise the steps of:

a) adding tetraborate to a relatively impure solution of transferrin to provide a solution of transferrin and tetraborate at a ratio of at least 1 mole tetraborate to 1 mole transferrin, for example at least 20 mole tetraborate to 1 mole transferrin;

b) applying the solution of transferrin and tetraborate to an anionic chromatographic material for which the transferrin has no specific binding affinity such that the transferrin- binds to the material;

c) washing the chromatographic material with a tetraborate solution of from 0.1 mM to 30 mM, for example 0.1 mM to 20 mM;

d) subsequently washing the chromatographic material with a tetraborate solution of from 5 mM to 60 mM, for example 30 to 60 mM or at least 30 mM ; and

e) optionally, recovering the transferrin from the anionic chromatographic material.

The term 'no specific binding activity' means that, preferably, the transferrin does not bind to the chromatographic material in an antibody-antigen binding manner.

A surprising advantage of adding tetraborate to the transferrin solution prior to loading the transferrin on to the anionic chromatography material is that this increases the load capacity of the material for transferrin. The load capacity may be increased by 2-fold, 3-fold or more. Thus more transferrin may be loaded on a given amount of matrix, and/or the amount of transferrin lost in the flow-through may be reduced. Preferably, tetraborate is added at a ratio from 1 mole tetraborate to 1 mole transferrin to 1000 mole tetraborate to 1 mole transferrin, for example at, from or up to at or about 1, 10, 15, 20, 30, 50, 100, 200, 300, 400, 450, 500, 600, 700, 800, 900 or 1000 mol.mol$^{-1}$. A preferred ratio is from 20 to 1000 mole tetraborate to 1 mole transferrin. The advantage of adding tetraborate to transferrin prior to loading the transferrin onto the anionic chromatographic material may be exploited alone or in combination with the wash step(s) of the purification process disclosed above. For example, tetraborate may be used to increase the transferrin-binding capacity of an anionic exchange matrix independently of or in combination with a purification process that uses that matrix.

The tetraborate solution used in the washing of step (c) may be at a concentration of from at or about 0.1 mM to at or about 30 mM. For example, the tetraborate solution used in (c) may be at a concentration from at or about 0.1 mM, 0.5 mM, 1 mM, 2 mM, 4 mM, 6 mM, 8 mM, 10 mM, 15 mM, 20 mM or 25 mM to at or about 0.5 mM, 1 mM, 2 mM, 4 mM, 6 mM, 8 mM, 10 mM, 15 mM, 20 mM, 25 mM or 30 mM. More particularly the tetraborate solution of (c) may be at a concentration from at or about 2.5 mM to at or about 7.5 mM, most particularly at or about 5 mM.

The tetraborate solution used in the washing of step (d) may be at a concentration from at or about 5 mM to at or about 60 mM. For example, the tetraborate solution used in (d) may be at a concentration from at or about 5 mM, 10 mM, 15 mM, 20 mM, 25 mM, 30 mM, 35 mM, 40 mM, 45 mM, 50 mM or 55 mM to at or about 10 mM, 15 mM, 20 mM, 25 mM, 30 mM, 35 mM, 40 mM, 45 mM, 50 mM, 55 mM or 60 mM. More particularly the tetraborate solution of (d) may be at a concentration from at or about 20 mM to at or about 40 mM, most particularly at or about 30 mM.

A surprising advantage of carrying out a low concentration 'pre-wash', such as the wash of step (c), prior to a higher concentration wash, such as the wash of step (d), is that this regime reduces the loss of transferrin during the higher concentration wash. Preferably, 'concentration' is 'tetraborate concentration'.

The tetraborate may be a tetraborate of a Group I metal, such as potassium tetraborate or sodium tetraborate. A preferred tetraborate is potassium tetraborate.

The transferrin may be recovered from the anionic chromatographic material by elution, for example by a change in pH and/or an increase in conductivity. The elution solution may have a pH of less than or equal to pH 5.2 and/or a conductivity of greater than or equal to 2.5 mS.cm$^{-1}$. An alternative elution liquid comprises 110 mM potassium tetraborate or sodium tetraborate.

Preferably the anionic chromatographic material comprises a functional ligand which is a strong anion exchanger or a weak anion exchanger. A functional ligand may be an ion. Examples of strong anion exchangers include quaternary amine groups ("Q"), *i.e.* -N$^+$(CH$_3$)$_3$ such as Q Sepharose Fast Flow (Q-FF) and Capto Q (both available from GE Healthcare, Little Chalfont, UK), UNOsphere™ Q (Bio-Rad, Hemel Hempstead, UK), Toyopearl® SuperQ (TosoH Corporation, Tokyo, Japan), Q Ceramic HyperD® (Pall, Portsmouth, UK) and POROS® 50 HQ (Applied Biosystems Inc., Foster City, CA, USA). Examples of weak anion exchangers include diethylaminoalkyl groups such as diethylaminoethyl groups ("DEAE", also referred to as "DE") i.e. -N$^+$H(CH$_2$CH$_3$)$_2$ such as DEAE Sepharose Fast Flow (DE-FF; available from GE Healthcare). Further strong and weak anion exchangers are described in 'Liquid column chromatography: a survey of modern techniques and applications' (1975, pages 344-351, Editors: Zdenĕk Deyl, Karel Macek, Jaroslav Janák, Publisher: Elsevier) and in 'Protein liquid chromatography' (2000, pages 18-21, Editor: Michael Kastener, Publisher: Elsevier) both of which are hereby incorporated by reference.

Weak anion exchangers may benefit from the use of wash buffers for steps (c) and (d) which are of a lower concentration than those used for strong anion exchangers.

Preferably the transferrin solution is loaded onto the anionic chromatography material at from about 0.01 mg transferrin per mL chromatography matrix to about 100 mg.mL$^{-1}$., for example about from at or about 0.01, 0.1, 1, 10, 20, 30, 40, 50, 60, 70, 80 or 90 mg.mL$^{-1}$ to at or about 0.1, 1, 10, 20, 30, 40, 50, 60, 70, 80, 90 or 100 mg.mL$^{-1}$. Preferred loading parameters are from at or about 10 to at or about 100 mg.mL$^{-1}$. A particularly preferred loading parameter is at or about 30 mg.mL$^{-1}$.

Preferably the transferrin solution which is loaded onto the anionic chromatography matrix has a pH of from about pH 8 to 10, such as from about pH 8.0 to 10.0 more preferably at, to or from pH 9.0 to 9.4.

Prior to loading the transferrin-containing material onto the anionic chromatographic material, the anionic chromatographic material may be equilibrated for example with one or more strengths of tetraborate solution, for example 110 mM potassium tetraborate and/or 30 mM potassium tetraborate. Potassium tetraborate may be partially or fully replaced by a different tetraborate such as a Group I metal tetraborate, such as sodium tetraborate.

A second aspect of the invention provides a process for purifying a transferrin solution (the transferrin solution may be considered to be a 'starting material'), the process comprising a cationic chromatographic step comprising the steps of:

a) applying a relatively impure solution of a transferrin to a cationic chromatographic material for which the transferrin has no specific binding affinity such that the transferrin binds to the material;
b) washing the chromatographic material with a wash buffer having a pH of from about 4 to about 5 and a salt concentration of 200 mM or less;
c) subsequently washing the chromatographic material with a wash buffer having a pH from about 4 to about 5 and a salt concentration of from about 50 mM to about 5 M; and
d) optionally, recovering the transferrin from the cationic chromatographic material.

Preferably the pH of the wash buffer of step (b) of the cationic chromatographic step is from at or about 4.0, 4.1, 4.2, 4.3, 4.4, 4.5, 4.6, 4.7, 4.8 or 4.9 to at or about 4.1, 4.2, 4.3, 4.4, 4.5, 4.6, 4.7, 4.8, 4.9 or 5.0, most preferably at or about pH 4.5.

Preferably the wash buffer of step (b) of the cationic chromatographic step has a total salt concentration of from about 10 mM to about 200 mM, more preferably at about 50 mM. More preferably the salt which provides the buffering activity of the wash buffer of step (b) of the cationic chromatographic step is present at a concentration of from about 10 mM to about 200 mM, more preferably at about 50 mM. Preferably the wash buffer of step (b) is substantially free of salt, other than the salt providing the buffering effect (i.e. the 'buffering salt') and most preferably is free of salt other than the buffering salt. The term 'substantially free' means that the level of salt, other than buffering salt, is undetectable and or is sufficiently low as to have no effect on the wash buffer relative to a wash buffer that is completely free of salt other than the buffering salt. For example, it is preferred that the wash buffer of step (b) is substantially free of sodium chloride.

It is preferred that the pH of the wash buffer of step (c) of the cationic chromatographic step is at or about 4.0, 4.1, 4.2, 4.3, 4.4, 4.5, 4.6, 4.7, 4.8 or 4.9 to at or about 4.1, 4.2, 4.3, 4.4, 4.5, 4.6, 4.7, 4.8, 4.9 or 5.0, most preferably at or about pH 4.5.

Preferably the wash buffer of step (c) of the cationic chromatographic step has a salt concentration of from about 50 mM to about 5 M, for example from at or about 50 mM, 100 mM, 150 mM, 200 mM, 250 mM, 500 mM, 1 M, 1.25 M, 1.5 M, 1.75 M, 2 M, 2.5 M, 3 M, 3.5 M, 4 M or 4.5 M to at or about 100 mM, 150 mM, 200 mM, 250 mM, 500 mM, 1 M, 1.25 M, 1.5 M, 1.75 M, 2 M, 2.5 M, 3 M, 3.5 M, 4 M, 4.5 M or 5 M, more preferably from at or about 250 mM to at or about 2 M, most preferably at or about 2 M. It is preferred that the wash buffer of step (c) has a higher salt concentration that the wash buffer of step (b). Example of 'higher' include by greater than or equal to 5, 10, 20, 30, 40, 50, 100, 150 mM.

Preferred wash buffers of step (c) of the cationic chromatographic step provide a recovery of at least 60, 70, 80, 90, 95 or 100% and/or a host cell protein (HCP) clearance (e.g. yeast antigen clearance) of at least 200, 225, 250, 275, 300, 325, 350, 375 or 400-fold. Particularly preferred wash buffers provide a recovery of at least 60, 70, 80, 90, 95 or 100% and a HCP (e.g. YA) clearance of at least 200, 225, 250, 275, 300, 325, 350, 375 or 400 and are defined by, that is satisfies, one or more (preferably both) of the following equations:

$$\text{Recovery} = -940.50069 + (441.61018 \times p) + (0.043309 \times s) - (47.57735 \times p^2) - (0.010263 \times p \times s)$$

$$\text{HCP (e.g. YA) clearance} = 16388.65744 - (10512.30681 \times p) - (1.70101 \times s) + (2257.17601 \times p^2) + (0.78628 \times p \times s) - (159.06573 \times p^3) - (0.090182 \times p^2 \times s)$$

In the above equations, p = pH and s = salt concentration in mM and recovery is % recovery (for example weight/weight).

For example, the wash buffer may provide a recovery of at least 60% and a HCP (e.g. YA) clearance of at least 275-fold. Parameters of a wash buffer providing a recovery of at least 60% and a HCP (e.g. YA) clearance of at least 275-fold may be defined by the following equations:

$$\text{Recovery (\%): } 60 = -940.50069 + (441.61018 \times p) + (0.043309 \times s) - (47.57735 \times p^2) - (0.010263 \times p \times s)$$

$$\text{HCP (e.g. YA) clearance (fold): } 275 = 16388.65744 - (10512.30681 \times p) - (1.70101 \times s) + (2257.17601 \times p^2) + (0.78628 \times p \times s) - (159.06573 \times p^3) - (0.090182 \times p^2 \times s)$$

In the above equations, p pH and s = salt concentration in mM.

Recovery (%) may be weight/weight. The term 'host cell protein' is well known to the skilled person. Host cell proteins include proteins native to and produced by a host cell which may be present as a contaminant in a protein, such as a heterologous protein, produced by the host cell. It is desirable to reduce, or eliminate, the amount of host cell protein present in a product produced from that host cell. For a yeast host cell, the host cell proteins may be known as 'yeast antigens'.

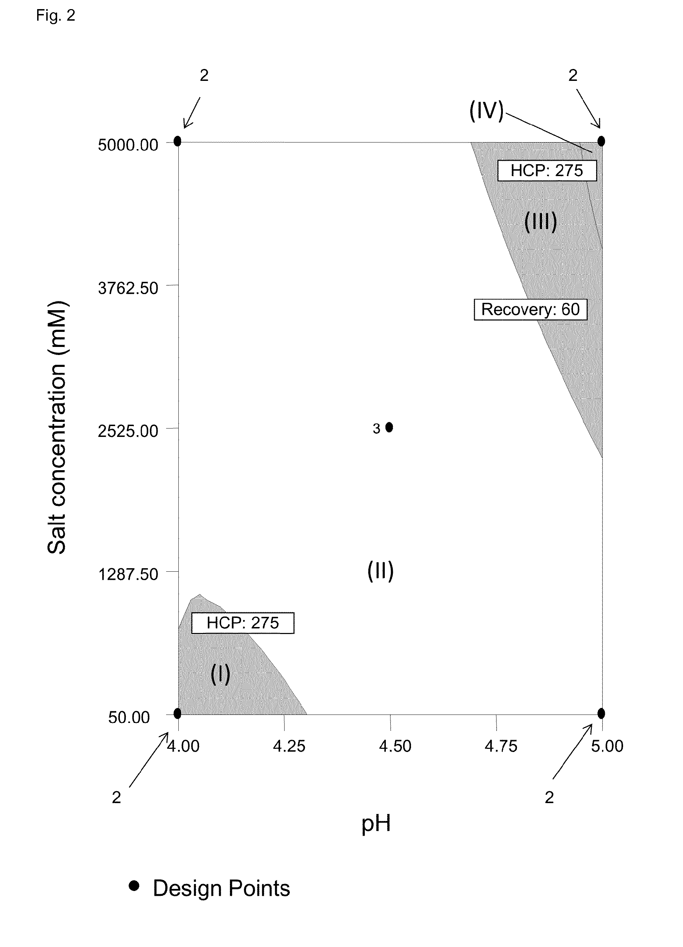

The pH, salt concentration, HCP (e.g. YA) clearance and recovery may be plotted on a graph, e.g. a an overlay plot. Fig.2 shows an example of an overlay plot. Such a graph gives a view of the 'design space'. The design space represents the combinations of pHs and salt concentrations at which the desired recovery (%) and HCP (e.g. YA) clearance (fold) is achieved. A design space can be generated ('defined') for any combination of desired recovery and HCP (YA) clearance.

The design space is the most accurate definition of the combinations of pHs and salt concentrations which provide the desired recovery and HCP (e.g. YA) clearance. However, the combinations may also be approximated by describing at least a part, and preferably a significant part, of the design space by one or more rectangular areas. For example, the design space defined by a recovery of 60% or more and a HCP (e.g. YA) clearance of 275-fold or more may be approximated by one or more of (i) pH 4.3 - 5.0, 50 - 1100mM salt, (ii) pH 4.0 - 4.7, 1100 - 5000mM salt, (iii) pH 4.7 - 5.0, 1100 - 2270mM salt (iv) pH 4.7 - 4.8, 2270 - 3900mM salt, (v) pH 4.8 - 4.9, 2270 - 2990mM salt and (vi) pH 4.7 - 4.75, 3900 - 4345mM salt. Preferably the design is approximated by (i) to (iv), more preferably by (i) to (v) and most preferably by (i) to (vi).Suitable salts include monovalent and divalent metal and ammonium salts. One preferred salt for the cationic chromatographic step is NaCl.

The wash buffer of step (c) may or may not comprise two or more salts. For example, the wash buffer of step (c) may or may not comprise sodium acetate and NaCl. Sodium acetate may be present at or about 27 mM.

A surprising advantage of low pH/high salt wash, such as those described above, is that this improves HCP clearance, e.g. yeast antigen clearance, across the cationic matrix without significantly lowering the recovery of transferrin from the matrix. This is in direct contrast to the expectation that use of such a high salt wash would result in an undesirably high loss of transferrin from the matrix.

The transferrin may be recovered from the cationic chromatographic material by elution, for example with a change of pH and/or conductivity and/or the addition of a specific eluent. The elution solution may comprise sodium phosphate and or sodium chloride. For example sodium phosphate may be present at or about 50 mM. Sodium chloride may be present at or about 150 mM. The pH of the elution solution may be at or about pH 6, for example at or about pH 6.0.

The cationic chromatographic step may comprise a further, for example a third wash, prior to recovery of the transferrin. Preferably the further wash step is carried out after the wash step of step (c). Preferably, the further wash step is carried out immediately after the wash step of step (c), *e.g.* with no intervening step. The further wash may be carried out using a wash buffer of 50 mM sodium acetate pH 5.0 to 5.2 (preferably pH 5.1) preferably with a conductivity of from 2.5 to 3.1 mS.cm$^{-1}$.

Preferably the transferrin solution is loaded onto the cationic chromatography material at up to 100 mg transferrin per mL chromatographic material, for example from at or about 10, 20, 30, 40, 50, 60, 70, 80 or 90 mg.mL$^{-1}$ to at or about 20, 30, 40, 50, 60, 70, 80, 90 or 100 mg.mL$^{-1}$ more preferably at from about 30 to about 50 mg.mL$^{-1}$.

Preferably the cationic chromatographic material comprises a functional ligand which is a strong cation exchanger. Examples of strong cation exchangers include a sulphonic group, *i.e.* -SO$_3^-$ *e.g.* SP Sepharose Fast Flow (SP-FF) and S-Spheradex; (both available from GE Healthcare), SP-Spherosil, SE-Cellulose A particularly preferred cationic chromatographic material is SP Sepharose Fast Flow. The cationic chromatographic material may comprise a functional ligand which is a weak cation exchanger, such materials include, CM-Sepharose FF or CM-Cellulose. Further strong and weak cation exchangers are described in 'Liquid column chromatography: a survey of modern techniques and applications' (1975, pages 344-351, Editors: Zdeněk Deyl, Karel Macek, Jaroslav Janák, Publisher: Elsevier) and in 'Protein liquid chromatography' (2000, pages 18-21, Editor: Michael Kastener, Publisher: Elsevier).

Prior to loading (for example, step (a)), the cationic chromatographic material may be equilibrated for example with 50 mM sodium acetate (from at or about pH 4 to pH 6, such as at or about pH 4.0 to pH 6.0, for example from at or about pH 5.0 to at or about pH 5.2, preferably at or about pH 5.1 and with a conductivity of from at or about 2.5 mS.cm$^{-1}$ to at or about 3.1 mS.cm$^{-1}$).

Following recovery of the transferrin, the cationic and/or anionic chromatographic material may be cleaned prior to storage and/or re-use of the material. Techniques for cleaning chromatographic materials are known in the art.

A third aspect of the invention provides a process for purifying a transferrin solution, the process comprising a cationic step according to the second aspect of the invention and an anionic chromatographic step according to the first aspect of the invention. Preferably, the cationic step is followed by the anionic step. Alternatively, the cationic step may follow the anionic step. Preferably, the cationic is immediately followed by the anionic step, *e.g.* with no intervening step. Optionally, other steps may be carried out in between the cationic and anionic steps. The eluate of the cationic step may or may not be diluted prior to being subjected to the anionic step.

Preferably the transferrin solution which is subjected to the purification of the invention is substantially, or completely, free of particles such as cells and cell debris. 'Substantially free' means that any particles present in the solution do not have a significant adverse effect on the purification process. Preferably, any particles present do not reduce the yield of the purification process by more than 10, 9, 8, 7, 6, 5, 4, 3, 2, 1, 0.5 or 0.1 % relative to a solution being completely free of particles.

The final transferrin-containing solution obtained from the process of the first, second or third aspect of the invention may or may not be subjected to one or more further steps such as purification steps. Such further steps may be carried out prior to one or more of the steps of the first, second or third aspects of the invention. For example, the first and second aspects of the invention may be separated by one or more of the further steps.

The one or more further purification steps may be selected from: anion exchange chromatography, cation exchange chromatography, buffer exchange, concentration, dilution, removal of metal ions such as iron ions, dialysis, diafiltration, pH-adjustment, (for example with solution comprising tetraborate and a base, such as potassium tetraborate and sodium hydroxide, particularly 250 mM potassium tetraborate and 0.5 M sodium hydroxide), treatment with a reducing agent, discolouration treatment (*e.g.* with charcoal) for example to remove yeast-derived and/or matrix-derived colourants, heating (including sterilisation), cooling, conditioning, ultrafiltration, cell separation techniques, such as centrifugation, filtration (*e.g.* cross-flow filtration, expanded bed chromatography and the like) methods of cell breakage, including beadmilling, sonication, enzymatic exposure and the like, ammonium sulphate precipitation, ethanol precipitation, acid extraction, solvent extraction, phosphocellulose chromatography, hydrophobic interaction chromatography, affinity chromatography, hydroxyapatite chromatography, lectin chromatography, metal affinity/chelating chromatography, high performance liquid chromatography ("HPLC"), reverse phase HPLC, conductivity adjustment, alkaline precipitation of the transferrin to a lipophilic phase and the like. Ultrafiltration is particularly preferred. 'Conditioning' includes adjusting one or more of pH and conductivity and/or the addition of solid and/or ingredients. Ultrafiltration may use a nominal molecular weight cut off of 10 000. Transferrin recovered from the process of the first, second or third aspect of the invention may be pH adjusted to from pH 6.7 to 7.3, for example by the addition of acetic acid. The pH-adjusted transferrin may then be concentrated and/or diafiltered against a NaCl solution. At the end of diafiltration the retentate may be further concentrated.

Any one or more of the above mentioned techniques may or may not be used to further purify the thus isolated protein to a commercially or industrially acceptable level of purity. By commercially or industrially acceptable level of purity, we include the provision of the transferrin protein in which other material (for example, one or more contaminants) are present at a level of less than 50 %, 40 %, 30 %, 20 %, 10 %, 5 %, 4 %, 3 %, 2 %, 1 %, 0.5 %, 0.1 %, 0.01 %, 0.001 %, 0.0001 %, 0.00001 %, or 0.000001 % and, most preferably at a level of 0 %.

A commercially or industrially acceptable level of purity may be obtained by a relatively crude purification method by which the protein product of choice is put into a form suitable for its intended purpose. A protein preparation that has been purified to a commercially or industrially acceptable level of purity may, in addition to the protein product of choice, also comprise, for example, cell culture components such as host cells or debris derived therefrom. Alternatively, high molecular weight components (such as host cells or debris derived therefrom) may or may not be removed (such as by filtration or centrifugation) to obtain a composition comprising the protein product of choice and, optionally, a functionally acceptable level of low molecular weight contaminants derived from the cell culture process.

The protein may or may not be purified to achieve a pharmaceutically acceptable level of purity. A protein has a pharmaceutically acceptable level of purity if it is essentially pyrogen free and can be used for its intended purpose and hence be administered in a pharmaceutically efficacious amount without causing medical effects not associated with the activity of the protein.

The transferrin product may be provided at a concentration of at least $10^{-4}$ g.L$^{-1}$, $10^{-3}$ g.L$^{-1}$, 0.01 g.L$^{-1}$, 0.02 g.L$^{-1}$, 0.03 g.L$^{-1}$, 0.04 g.L$^{-1}$, 0.05 g.L$^{-1}$, 0.06 g.L$^{-1}$,0.07 g.L$^{-1}$, 0.08 g.L$^{-1}$, 0.09 g.L$^{-1}$, 0.1 g.L$^{-1}$, 0.2 g.L$^{-1}$, 0.3 g.L$^{-1}$, 0.4 g.L$^{-1}$, 0.5 g.L$^{-1}$, 0.6 g.L$^{-1}$, 0.7 g.L$^{-1}$, 0.8 g.L$^{-1}$, 0.9 g.L$^{-1}$, 1 g.L$^{-1}$, 2 g.L$^{-1}$, 3 g.L$^{-1}$, 4 g.L$^{-1}$, 5 g.L$^{-1}$, 6 g.L$^{-1}$, 7 g.L$^{-1}$, 8 g.L$^{-1}$, 9 g.L$^{-1}$, 10 g.L$^{-1}$, 15 g.L$^{-1}$, 20 g.L$^{-1}$, 25 g.L$^{-1}$ 1, 30 g.L$^{-1}$, 40 gL$^{-1}$,50 g.L$^{-1}$, 60 g.L$^{-1}$, 70 g.L$^{-1}$, 70 g.L$^{-1}$, 90 g.L$^{-1}$, 100 g.L$^{-1}$, 150 g.L$^{-1}$, 200 g.L$^{-1}$, 250 g.L$^{-1}$, 300 g.L$^{-1}$, 350 g.L$^{-1}$, 400 g.L$^{-1}$, 500 g.L$^{-1}$, 600 g.L$^{-1}$, 700 g.L$^{-1}$, 800 g.L$^{-1}$, 900 g.L$^{-1}$, 1000 g.L$^{-}$.

The transferrin-containing solution obtained by the process of the first, second or third aspect of the invention may be further purified and/or formulated for intravenous administration to a human and/or for provision as a cell culture ingredient and/or sterilised and/or placed into a final container.

It is preferred that the transferrin-containing solution obtained by the first, second or third aspect of the invention is suspended in a liquid, for example a solution of a buffer, suitable for final use of the transferrin. Such solutions include sodium chloride solutions, for example 145 mM NaCl.

The process of the first, second or third aspects of the invention may be preceded by one or more of the following steps: fermentation, primary separation, centrate conditioning, affinity separation, metal chelating separation, hydrophobic separation, liquid/liquid extraction, precipitation, ultrafiltration, size exclusion separation and/or mixed mode separation.

The transferrin may or may not be holoized prior to and/or following the cationic chromatographic step and/or the anionic chromatographic step of the first, second or third aspects of the invention. The transferrin may or may not be apoized prior to and/or following the cationic chromatographic step and/or the anionic chromatographic step of the first, second or third aspects of the invention. That is, the transferrin which is subjected to the purification process may be apo-transferrin, partially iron-saturated transferrin or holo-transferrin.

Holoization and apoization techniques are known in the art. Transferrin may be holoized by the addition of sodium bicarbonate and iron (for example in the form of ammonium iron citrate) to about 2 moles of $Fe^{3+}$ per mole of transferrin and incubating at ambient temperature (*e.g.* about 20 to 25 °C) for at least one hour. Transferrin may be apoized at low pH in the presence of a chelating agent. For example transferrin may be apoized by the addition of 1 part transferrin solution to 9 parts of apoization solution and incubating the resultant mixture, with stirring, overnight at about 4 °C. Apoization solution may comprise 100 mM sodium acetate, 100 mM sodium citrate, 10 mM EDTA at pH 4.5.

The relatively impure transferrin may be obtained from a blood source or from a recombinant source such as prokaryotic (*e.g.* bacterial) or eukaryotic (*e.g.* fungal, animal, insect or plant). A preferred source of the relatively impure solution of transferrin is a microbial cell, such as a fungal cell (preferably a yeast cell such as *Saccharomyces* (*e.g.* S. *cerevisiae*), *Pichia* (*e.g. P. pastoris*) *or Kluyveromyces* (*e.g. K. lactis*)) or a bacterial cell (*e.g. a Bacillus* or *Escherichia coli*), a mammalian cell (*e.g.* Chinese Hamster Ovary (CHO) or Baby Hamster Kidney (BHK) cell) or from blood or a blood fraction such as serum. The blood or blood fraction may be human or animal derived (such as bovine derived).

The 'transferrin' of the invention may be any protein with iron binding capacity which belongs to the transferrin family as described, *e.g.*, by Lambert et al., Comparative Biochemistry and Physiology, Part B, 142 (2005), 129-141, and by Testa, Proteins of iron metabolism, CRC Press, 2002; Harris & Aisen, Iron carriers and iron proteins, Vol. 5, Physical Bioinorganic Chemistry, VCH, 1991.

Examples of transferrin family proteins are serum transferrin, ovotransferrin, melanotransferrin and lactoferrin and their derivatives and variants, such as mutant transferrins (Mason et al., (1993) Biochemistry, 32, 5472; Mason et al., (1998), Biochem. J., 330, 35), truncated transferrins, transferrin lobes (Mason et al., (1996) Protein Expr. Purif., 8, 119; Mason et al., (1991) Protein Expr. Purif., 2, 214), mutant lactoferrins, truncated lactoferrins, lactoferrin lobes, mutant ovotransferrin, truncated ovotransferrin, melanotransferrin lobes, truncated melanotransferrin or fusions of any of the above to other peptides, polypeptides or proteins (Shin et al., (1995) Proc. Natl. Acad. Sci. USA, 92, 2820; Ali et al., (1999) J. Biol. Chem., 274, 24066; Mason et al., (2002) Biochemistry, 41, 9448). Serum transferrins are preferred, particularly a human serum transferrin (HST) having the amino acid sequence of SEQ ID NO: 1 with 679 amino acids, also called the C1 variant (Accession number NP_001054). Lactoferrins are preferred, particularly a human lactoferrin having the amino acid sequence of SEQ ID NO: 2 with 691 amino acids. Melanotransferrins are preferred, particularly human melanotransferrin having the amino acid sequence residue 20-738 of SEQ ID NO: 3.

The term 'transferrin' also includes both natural and engineered variants of transferrins for example fusions and conjugations of transferrins and conjugation-competent transferrins such as 'thio-transferrins'. Examples are described in WO2008/152140 and PCT/EP2008/060482, both of which are hereby incorporated by reference. Conjugations of transferrin, for example 'thio-transferrins' may be prepared according to PCT/EP2008/060482 and "Protein Formulation and Delivery", E. J. McNally (Ed.), published by Marcel Dekker Inc. New York 2000 and "Rational Design of Stable Protein Formulations - Theory and Practice"; J. F. Carpenter and M. C. Manning (Ed.) Pharmaceutical Biotechnology Vol 13. Kluwer Academic/Plenum Publishers, New York 2002, Yazdi and Murphy, (1994) Cancer Research 54, 6387-6394, Widera et al., (2003) Pharmaceutical Research 20, 1231-1238; Lee et al., (2005) Arch. Pharm. Res. 28, 722-729. The transferrin of or purified by the claimed invention may or may not be a natural or engineered variant of transferrin as described herein. For example, the transferrin may or may not have one or more thiol groups available for conjugation to another molecule.

[SEQ ID No. 1]

```
VPDKTVRWCA  VSEHEATKCQ  SFRDHMKSVI  PSDGPSVACV  KKASYLDCIR  AIAANEADAV         60
TLDAGLVYDA  YLAPNNLKPV  VAEFYGSKED  PQTFYYAVAV  VKKDSGFQMN  QLRGKKSCHT        120
GLGRSAGWNI  PIGLLYCDLP  EPRKPLEKAV  ANFFSGSCAP  CADGTDFPQL  CQLCPGCGCS        180
TLNQYFGYSG  AFKCLKDGAG  DVAFVKHSTI  FENLANKADR  DQYELLCLDN  TRKPVDEYKD        240
CHLAQVPSHT  VVARSMGGKE  DLIWELLNQA  QEHFGKDKSK  EFQLFSSPHG  KDLLFKDSAH        300
GFLKVPPRMD  AKMYLGYEYV  TAIRNLREGT  CPEAPTDECK  PVKWCALSHH  ERLKCDEWSV        360
NSVGKIECVS  AETTEDCIAK  IMNGEADAMS  LDGGFVYIAG  KCGLVPVLAE  NYNKSDNCED        420
TPEAGYFAVA  VVKKSASDLT  WDNLKGKKSC  HTAVGRTAGW  NIPMGLLYNK  INHCRFDEFF        480
SEGCAPGSKK  DSSLCKLCMG  SGLNLCEPNN  KEGYYGYTGA  FRCLVEKGDV  AFVKHQTVPQ        540
NTGGKNPDPW  AKNLNEKDYE  LLCLDGTRKP  VEEYANCHLA  RAPNHAVVTR  KDKEACVHKI        600
LRQQQHLFGS  NVTDCSGNFC  LFRSETKDLL  FRDDTVCLAK  LHDRNTYEKY  LGEEYVKAVG        660
NLRKCSTSSL  LEACTFRRP                                                        679
```

SEQ ID No. 2]

```
GRRRSVQWCA  VSQPEATKCF  QWQRNMRKVR  GPPVSCIKRD  SPIQCIQAIA  ENRADAVTLD         60
GGFIYEAGLA  PYKLRPVAAE  VYGTERQPRT  HYYAVAVVKK  GGSFQLNELQ  GLKSCHTGLR        120
RTAGWNVPIG  TLRPFLNWAG  PPEPIEAAVA  RFFSASCVPG  ADKGQFPNLC  RLCAGTGENK        180

CAFSSQEPYF  SYSGAFKCLR  DGAGDVAFIR  ESTVFEDLSD  EAERDEYELL  CPDNTRKPVD        240
KFKDCHLARV  PSHAVVARSV  NGKEDAIWNL  LRQAQEKFGK  DKSPKFQLFG  SPSGQKDLLF        300
KDSAIGFSRV  PPRIDSGLYL  GSGYFTAIQN  LRKSEEEVAA  RRARVVWCAV  GEQELRKCNQ        360
WSGLSEGSVT  CSSASTTEDC  IALVLKGEAD  AMSLDGGYVY  TAGKCGLVPV  LAENYKSQQS        420
SDPDPNCVDR  PVEGYLAVAV  VRRSDTSLTW  NSVKGKKSCH  TAVDRTAGWN  IPMGLLFNQA        480
GSCKFDEYFS  QSCAPGSDPR  SNLCALCIGD  EQGENKCVPN  SNERYYGYTG  AFRCLAENAG        540
DVAFVKDVTV  LQNTDGNNNE  AWAKDLKLAD  FALLCLDGKR  KPVTEARSCH  LAMAPNHAVV        600
SRMDKVERLK  QVLLHQQAKF  GRNGADCPDK  FCLFQSETKN  LLFNDNTECL  ARLHGKTTYE        660
KYLGPQYVAG  ITNLKKCSTS  PLLEACEFLR  K                                        691
```

[SEQ ID No. 3]

```
MRGPSGALWL LLALRTVLGG MEVRWCATSD PEQHKCGNMS EAFREAGIQP SLLCVRGTSA        60
DHCVQLIAAQ EADAITLDGG AIYEAGKEHG LKPVVGEVYD QEVGTSYYAV AVVRRSSHVT       120
IDTLKGVKSC HTGINRTVGW NVPVGYLVES GRLSVMGCDV LKAVSDYFGG SCVPGAGETS       180
YSESLCRLCR GDSSGEGVCD KSPLERYYDY SGAFRCLAEG AGDVAFVKHS TVLENTDGKT       240
LPSWGQALLS QDFELLCRDG SRADVTEWRQ CHLARVPAHA VVVRADTDGG LIFRLLNEGQ       300
RLFSHEGSSF QMFSSEAYGQ KDLLFKDSTS ELVPIATQTY EAWLGHEYLH AMKGLLCDPN       360
RLPPYLRWCV LSTPEIQKCG DMAVAFRRQR LKPEIQCVSA KSPQHCMERI QAEQVDAVTL       420
SGEDIYTAGK TYGLVPAAGE HYAPEDSSNS YYVVAVVRRD SSHAFTLDEL RGKRSCHAGF       480
GSPAGWDVPV GALIQRGFIR PKDCDVLTAV SEFFNASCVP VNNPKNYPSS LCALCVGDEQ       540
GRNKCVGNSQ ERYYGYRGAF RCLVENAGDV AFVRHTTVFD NTNGHNSEPW AAELRSEDYE       600
LLCPNGARAE VSQFAACNLA QIPPHAVMVR PDTNIFTVYG LLDKAQDLFG DDHNKNGFKM       660
FDSSNYHGQD LLFKDATVRA VPVGEKTTYR GWLGLDYVAA LEGMSSQQCS GAAAPAPGAP       720
LLPLLLPALA ARLLPPAL                                                    738
```

A particularly preferred transferrin consists of or comprises the amino acid sequence of SEQ ID No. 4:

[SEQ ID No. 4]

```
VPDKTVRWCA VSEHEATKCQ SFRDHMKSVI PSDGPSVACV KKASYLDCIR AIAANEADAV        60
TLDAGLVYDA YLAPNNLKPV VAEFYGSKED PQTFYYAVAV VKKDSGFQMN QLRGKKSCHT       120
GLGRSAGWNI PIGLLYCDLP EPRKPLEKAV ANFFSGSCAP CADGTDFPQL CQLCPGCGCS       180
TLNQYFGYSG AFKCLKDGAG DVAFVKHSTI FENLANKADR DQYELLCLDN TRKPVDEYKD       240
CHLAQVPSHT VVARSMGGKE DLIWELLNQA QEHFGKDKSK EFQLFSSPHG KDLLFKDSAH       300
GFLKVPPRMD AKMYLGYEYV TAIRNLREGT CPEAPTDECK PVKWCALSHH ERLKCDEWSV       360
NSVGKIECVS AETTEDCIAK IMNGEADAMS LDGGFVYIAG KCGLVPVLAE NYNKADNCED       420
TPEAGYFAVA VVKKSASDLT WDNLKGKKSC HTAVGRTAGW NIPMGLLYNK INHCRFDEFF       480
SEGCAPGSKK DSSLCKLCMG SGLNLCEPNN KEGYYGYTGA FRCLVEKGDV AFVKHQTVPQ       540
NTGGKNPDPW AKNLNEKDYE LLCLDGTRKP VEEYANCHLA RAPNHAVVTR KDKEACVHKI       600
LRQQQHLFGS NVADCSGNFC LFRSETKDLL FRDDTVCLAK LHDRNTYEKY LGEEYVKAVG       660
NLRKCSTSSL LEACTFRRP                                                   679
```

Preferably the transferrin which undergoes purification does not contain a leader sequence. For example, a transferrin may be produced with a leader sequence and the leader sequence may or may not be removed prior to the purification. Alternatively, the transferrin may be produced without the leader sequence. Examples of transferrin sequences without a leader sequence are given in SEQ ID No. 1 and SEQ ID No. 4.

The transferrin may be modified relative to a naturally occurring transferrin or to a known engineered variant of transferrin. For example, the transferrin may consist of or comprise an amino acid sequence which has at least 25% identity to SEQ ID NO: 1, SEQ ID No. 2 or SEQ ID No. 3 or SEQ ID No. 4, particularly at least 30%, 35%, 40%, 45%, 50%, 55%, 60%, 65%, 70%, 75%, 80%, 85%, 90%, 91 %, 92%, 93%, 94%, 95%, 96%, 97%, 98%, 99%, 99.1%, 99.2%, 99.3%, 99.4%, 99.5%, 99.6%, 99.7%, 99.8%, or 99.9%. More particularly, it may have 100% identity to SEQ ID No. 1, SEQ ID No. 2, SEQ ID No. 3 or most particularly SEQ ID No. 4.

The amino acid sequence of the transferrin family protein may have a length of at least 167 amino acids, particularly at least 300 amino acids, 630 amino acids, 691 amino acids, 694 amino acids or 695 amino acids. The length is typically at most 1274 amino acids, particularly at most 819 amino acids, at most 722 amino acids, at most 717 amino acids or at most 706 amino acids. The length may particularly be 679 amino acids. It may be a vertebrate transferrin with 691-717 amino acids or a mammalian transferrin with a length of 695-706 amino acids.

The transferrin family protein, particularly one having a length of 694-706 amino acids, generally contains two domains (called the N- and C-lobes), each having around 331-341 residues and each binding one atom of Fe (III) and one carbonate anion, connected by an inter-lobe linker of about 7 residues. Typically, each iron is coordinated to four conserved amino acid residues: one Asp, two Tyr and one His, and the carbonate anion is bound to an Arg and a Thr.

Preferably, the transferrin family protein purified in the invention has at least 40% or at least 60% identity to full-length HST (residues 1-679 of SEQ ID NO: 1) or to the C-lobe of HST (residues 339-679 of SEQ ID NO. 1). The primary receptor-recognition site of HST for the TfR is in the C-lobe, and proteolytically isolated HST C-lobe is able to deliver ferric iron to cells.

The human serum transferrin may be the variant designated $TfC_1$, $TfC_2$ or $TfC_3$.

A number of proteins are known to exist within the transferrin family and a non-exclusive list is shown below. The list indicates the full length of the sequences, including the mature protein and the leader sequence. Each of these transferrins, and variants thereof, may or may not be purified by the process of the invention.

**Table 1: Examples of transferrins**

| Protein | Species | Common Name | Accession Number | Identity to SEQ ID NO: 1 | Length |
|---|---|---|---|---|---|
| Transferrin | *Homo sapiens* | Human | NP_001054 | 100 % | 698 aa |
| Transferrin | *Canis lupus familiaris* | Dog | XP_864515 | 73.2 | 706 aa |
| Transferrin | *Mus musculus* | House mouse | NP_598738 | 73.7 | 697 aa |
| Transferrin | *Rattus norvegicus* | Norway rat | NP_001013128 | 73.6 | 698 aa |
| Transferrin | *Sus scrofa* | Pig | CAA30943 | 71.6 | 696 aa |
| Transferrin | *Oryctolagus cuniculus* | Rabbit | AAB94136 | 79.0 | 695 aa |
| Transferrin | *Equus caballus* | Horse | NP_001075415 | 73.7 | 706 aa |
| Transferrin | *Bos taurus* | Cattle | NP_803450 | 70.1 | 704 aa |
| Transferrin | *Gallus gallus* | Chicken | NP_990635 | 53.1 | 705 aa |
| Transferrin | *Marmota monax* | Woodchuck | AAP37129 | | 694 aa |
| Transferrin | *Xenopus laevis* | African clawed frog | NP_001079812 | | 717 aa |
| Transferrin | *Xenopus tropicalis* | African clawed frog | NP_001027487 | 49.6 | 703 aa |
| Transferrin | *Chamaeleo calyptratus* | Veiled Chameleons | CAK18229 | | 710 aa |
| Transferrin | *Lacerta agilis* | Sand Lizard | CAK18228 | | 714 aa |
| Transferrin | *Eublepharis macularius* | Leopard gecko | CAK18227 | | 703 aa |
| Transferrin | *Pogona vitticeps* | Central bearded dragon | CAK18226 | | 702 aa |
| Transferrin | *Anolis sagrei* | Brown anole | CAK18225 | | 710 aa |
| Transferrin | *Lamprophis fuliginosus* | African House Snake | CAK18223 | | 711 aa |
| Transferrin | *Natrix natrix* | Grass Snake | CAK18221 | 50.0 | 710 aa |
| Transferrin | *Oncorhynchus mykiss* | Rainbow trout | BAA84103 | | 691 aa |
| Transferrin | *Oryzias latipes* | Japanese medaka | BAA10901 | | 690 aa |

(continued)

| Protein | Species | Common Name | Accession Number | Identity to SEQ ID NO: 1 | Length |
|---|---|---|---|---|---|
| Transferrin | *Oreochromis niloticus* | Nile tilapia | ABB70391 | | 694 aa |
| Transferrin | *Oncorhynchus tshawytscha* | Chinook salmon | AAF03084 | | 672 aa |
| Transferrin | *Gadus morhua* | Atlantic cod | AAB08440 | | 642 aa |
| Transferrin | *Salmo trutta* | Brown trout | BAA84102 | | 691 aa |
| Transferrin | *Salvelinus namaycush* | Lake trout | BAA84101 | | 691 aa |
| Transferrin | *Salvelinus fontinalis* | Brook trout | BAA84100 | | 691 aa |
| Transferrin | *Salvelinus pluvius* | Japanese fish | BAA84099 | | 691 aa |
| Transferrin | *Oncorhynchus masou* | Cherry salmon | BAA84098 | | 691 aa |
| Transferrin | *Oncorhynchus rhodurus* | Amago | BAA84097 | | 691 aa |
| Transferrin | *Oncorhynchus nerka* | Sockeye salmon | BAA84096 | 50.2 | 691 aa |
| Transferrin | *Paralichthys olivaceus* | Bastard halibut | BAA28944 | | 685 aa |
| Transferrin | *Oncorhynchus kisutch* | Coho salmon | BAA13759 | | 687 aa |
| Transferrin | *Oreochromis aureus* | Tilapia (cichlid) | CAC59954 | | 167 aa |
| Transferrin | *Danio rerio* | Zebrafish | DAA01798 | | 675 aa |
| Transferrin | *Pagrus major* | Red seabream | AAP94279 | | 691 aa |
| Melanotransferrin | *Homo sapiens* | Human | NP_005920 | 45.7 | 738 aa |
| Meanotransferrin | *Canis lupus familiaris* | Dog | XP_545158 | 42.3 | 1193 aa |
| Melanotransferrin | *Mus musculus* | House mouse | NP_038928 | 44.4 | 738 aa |
| Melanotransferrin | *Rattus norvegicus* | Norway rat | XP_237839 | 44.7 | 738 aa |
| Melanotransferrin | *Gallus gallus* | Chicken | NP_990538 | 43.6 | 738 aa |
| Lactotransferrin | *H. sapiens* | Human | NP_002334 | 61.8 | 710 aa |
| Lactotransferrin | *Pan troglodytes* | Chimpanzee | XP_516417 | 62.0 | 711 aa |
| Lactotransferrin | *Canis lupus familiaris* | Dog | XP_864480 | 61.9 | 724 aa |
| Lactotransferrin | *Mus musculus* | House mouse | NP_032548 | 57.6 | 707 aa |
| Lactotransferrin | *Rattus norvegicus* | Norway rat | XP_236657 | 53.8 | 729 aa |
| Lactoferrin | *Sus scrofa* | Pig | AAA31059 | 61.1 | 703 aa |

(continued)

| Protein | Species | Common Name | Accession Number | Identity to SEQ ID NO: 1 | Length |
|---|---|---|---|---|---|
| Lactoferrin | *Camelus dromedarius* | Arabian camel | CAB53387 | 62.0 | 708 aa |
| Lactoferrin | *Equus caballus* | Horse | CAA09407 | 63.7 | 695 aa |
| Lactoferrin | *Bos taurus* | Cattle | AAA30610 | 62.0 | 708 aa |
| Lactoferrin | *Bubalus bubalis* | Water buffalo | CAA06441 | 62.1 | 708 aa |
| Lactoferrin | *Capra hircus* | Goat | ABD49106 | 62.2 | 708 aa |
| Lactoferrin | *Bos grunniens* | Domestic yak | ABD49105 | 62.0 | 708 aa |
| Lactoferrin | *Ovis aries* | Sheep | AAV92908 | 62.4 | 708 aa |
| Ovotransferrin | *Anas platyrhynchos* | Mallard duck | P56410 | 54.4 | 686 aa |
| Ovotransferrin | *Gallus gallus* | Chicken | CAA26040 | 53.1 | 705 aa |

The transferrin may or may not be fused and/or conjugated to a protein or other molecule such as a bioactive compound. The protein or other molecule, such as a bioactive compound, may or may not have a therapeutic and/or diagnostic use. In this context, 'fusion' includes a protein in which the transferrin and the other protein are encoded by the same DNA sequence whereas 'conjugation' includes a protein in which the transferrin has a protein or other molecule bound to it through any other means, for example through chemical conjugation. US 20030221201 and 20040023334 describe fusion proteins comprising a transferrin protein fused to a therapeutic protein. The transferrin may or may not be both a fused and conjugated transferrin.

The transferrin may or may not be a therapeutic or diagnostic transferrin. A 'therapeutic transferrin' is a transferrin which is suitable for use in preventative therapy or for curative therapy, for example treatment of anaemia. A 'diagnostic transferrin' is a transferrin which is suitable for use in diagnosis, for example the transferrin may be fused or conjugated to an antibody or to a label such as label or contrast agent suitable for use in imaging. The diagnostic transferrin may be used *in vivo* or *in vitro.*

The therapeutic transferrin may comprise a chemotherapy drug for use in cancer chemotherapy. It may be cytostatic or cytotoxic; it may be a tumor-inhibiting agent.

The bioactive compound may be a polypeptide (protein), particularly a recombinant protein pharmaceutical. It may be a chemotherapy or radiotherapy drug used to treat cancers and/or other related diseases.

The bioactive compound may be conjugated to a free thiol of a transferrin. The bioactive compound includes but is not limited to, peptides, polypeptides or proteins (either natural, recombinant, or synthetic). The free thiol may be on a cysteine residue.

A fourth aspect of the invention comprises a process for producing a transferrin comprising expressing a transferrin, for example in cells, particularly in fungal cells such as yeast cells, and purifying the resultant transferrin in accordance with the first, second, or third aspect of the invention.

A fifth aspect of the invention comprises the step of lyophilising the transferrin purified by the first, second, third or fourth aspect of the invention.

A sixth aspect of the invention provides a purified transferrin obtained or obtainable by a process according to the first, second, third, fourth or fifth aspect of the invention.

A seventh aspect of the invention relates to the use of a transferrin according to the first, second, third, fourth, fifth or sixth aspect of the invention in therapy (preventative and/or curative) or diagnosis. For example, the transferrin may be used in leukaemia treatment to prevent the harmful effects of non-transferrin-bound iron in a patient. The transferrin, for example a fused or conjugated transferrin, may be used in targeting, for example in diagnosis, imaging, localization and/or treatment of tumours.

An eighth aspect of the invention relates to the use of a transferrin according to the first, second, third, fourth, fifth, or sixth aspect of the invention in cell culture such as mammalian cell culture, particularly human cell culture. Cell culture may be at laboratory or industrial scale. The invention also relates to a cell culture medium comprising the transferrin according to the first, second, third, fourth, fifth or sixth aspect of the invention. The invention also includes manufacture of such a cell culture medium.

A ninth aspect of the invention provides a pharmaceutical composition comprising a transferrin purified according to the first, second, third, fourth or fifth aspect of the invention and a pharmaceutically acceptable carrier and/or diluent.

Therefore, the purified transferrin may be formulated with a carrier or diluent. The thus formulated transferrin may optionally be presented in a unit dosage form. The carrier(s) or diluent(s) must be "acceptable" in the sense of being compatible with the desired protein. Typically, the carriers or diluents will be water or saline which will be sterile and pyrogen free. The invention also includes provision of a transferrin according to the invention for use as a pharmaceutical ingredient. The transferrin may or may not be fused or conjugated to another molecule which may or may not be a bioactive molecule.

With regards the 'starting material' (*i.e.* the relatively impure transferrin solution from which the transferrin is purified by the process of the invention), the transferrin or fusions of transferrin and another protein or proteins can be prepared by methods know in the art (Sanker, (2004), Genetic Eng. News, 24, 22-28, Schmidt, (2004), Appl. Microbiol. Biotechnol., 65, 363-372) including but not limited to expression in mammalian cell culture (Mason et al., (2004), Protein Expr. Purif., 36, 318-326; Mason et al., (2002), Biochemistry, 41, 9448-9454) from cells lines such as CHO (and its variants), NS0, BHK, HEK293, Vero or PERC6 cells by transformation or transient expression; insect cell culture (Lim et al., (2004) Biotechnol. Prog., 20, 1192-1197); plant cell culture from such plants as Lemna; transgenic animals (Dyck et al., (2003) Trends in Biotechnology, 21, 394-399); transgenic plants (Ma et al., (2003) Nature Reviews Genetics, 4, 794-805); Gram positive and Gram negative bacteria such as *Bacillus* and *Escherichia coli* (Steinlein, and Ikeda, (1993), Enzyme Microb. Technol., 15, 193-199); filamentous fungi including but not restricted to *Aspergillus* spp (EP 238023, US 5,364,770, US 5,578,463, EP184438, EP284603, WO 2000/056900, WO9614413), *Trichoderma* spp and *Fusarium* spp (Navalainen et al., (2005), Trends in Biotechnology, 23, 468-473).

Polypeptides which are variants of full-length HST may be expressed recombinantly from baby hamster kidney (BHK) cells (Mason et al., (2004), Protein Expr. Purif., 36, 318-326, Mason et al., (2002), Biochemistry, 41, 9448-9454), *D. melanogaster* S2 cells (Lim et al., (2004), Biotechnol Prog., 20, 1192-1197) and as a non-N-linked glycosylated mutant from BHK cells (Mason et al., (2001), Protein Expr. Purif., 23, 142-150, Mason et al., (1993), Biochemistry, 32, 5472-5479) and *S. cerevisiae* (Sargent et al., (2006), Biometals 19, 513-519). The polypeptides which are variants of C-lobe of HST (NTf/2C) may be expressed from BHK cells (Mason et al., (1997), Biochem. J., 326 (Pt 1), 77-85). In one embodiment the host cell is a yeast cell, such as a member of the *Saccharomyces, Kluyveromyces,* or *Pichia* genus, such as *Sacharomyces cerevisiae, Kluyveromyces lactis, Pichia pastoris* (Mason et al., (1996), Protein Expr. Purif., 8, 119-125, Steinlein et al., 1995 Protein Expr. Purif., 6, 619-624), *Pichia methanolica* (Mayson et al., (2003) Biotechnol. Bioeng., 81, 291-298) and *Pichia membranaefaciens,* or *Zygosaccharomyces rouxii* (formerly classified as *Zygosaccharomyces bisporus), Zygosaccharomyces bailii, Zygosaccharomyces fermentati, Hansenula polymorpha* (also known as *Pichia angusta*) or *Kluyveromyces drosophilarum* are preferred.

The host cell may be any type of cell. The host cell may or may not be an animal (such as mammalian, avian, insect, etc.), plant, fungal or bacterial cell. Bacterial and fungal, such as yeast, host cells may or may not be preferred.

Typical prokaryotic vector plasmids are: pUC18, pUC19, pBR322 and pBR329 available from BioRad Laboratories (Richmond, CA, USA); pTrc99A, pKK223-3, pKK233-3, pDR540 and pRIT5 available from Pharmacia (Piscataway, NJ, USA); pBS vectors, Phagescript vectors, Bluescript vectors, pNH8A, pNH16A, pNH18A, pNH46A available from Stratagene Cloning Systems (La Jolla, CA 92037, USA).

A typical mammalian cell vector plasmid is pSVL available from Pharmacia (Piscataway, NJ, USA). This vector uses the SV40 late promoter to drive expression of cloned genes, the highest level of expression being found in T antigen-producing cells, such as COS-1 cells. An example of an inducible mammalian expression vector is pMSG, also available from Pharmacia (Piscataway, NJ, USA). This vector uses the glucocorticoid-inducible promoter of the mouse mammary tumour virus long terminal repeat to drive expression of the cloned gene.

Methods well known to those skilled in the art can be used to construct expression vectors containing the coding sequence of a desired protein and, for example appropriate transcriptional or translational controls. One such method involves ligation via cohesive ends. Compatible cohesive ends can be generated on the DNA fragment and vector by the action of suitable restriction enzymes. These ends will rapidly anneal through complementary base pairing and remaining nicks can be closed by the action of DNA ligase.

A further method uses synthetic double stranded oligonucleotide linkers and adaptors. DNA fragments with blunt ends are generated by bacteriophage T4 DNA polymerase or *E. coli* DNA polymerase I which remove protruding 3' termini and fill in recessed 3' ends. Synthetic linkers and pieces of blunt-ended double-stranded DNA which contain recognition sequences for defined restriction enzymes, can be ligated to blunt-ended DNA fragments by T4 DNA ligase. They are subsequently digested with appropriate restriction enzymes to create cohesive ends and ligated to an expression vector with compatible termini. Adaptors are also chemically synthesised DNA fragments which contain one blunt end used for ligation but which also possess one preformed cohesive end. Alternatively a DNA fragment or DNA fragments can be ligated together by the action of DNA ligase in the presence or absence of one or more synthetic double stranded oligonucleotides optionally containing cohesive ends.

Synthetic linkers containing a variety of restriction endonuclease sites are commercially available from a number of sources including Sigma-Genosys Ltd, London Road, Pampisford, Cambridge, United Kingdom.

The transferrin or fusions of transferrin and another protein or proteins will be expressed from a nucleotide sequence, which may or may not contain one or more introns. Additionally the nucleotide sequence may or may not be codon optimised for the host by methods known to the art.

The transferrin or fusions of transferrin and another protein or proteins can be expressed as variants with reduced N-linked glycosylation. Accordingly, in case of human serum transferrin (HST), N413 can be changed to any amino acid, preferably, Q, D, E or A; S415 can be changed to any amino acid except S or T, preferably, A; T613 can be changed to any amino acid except S or T, preferably, A; N611 can be changed to any amino acid; or combinations of the above. Where the transferrin is not HST, reduction in N-glycosylation can be achieved by similar modification to the protein primary sequence. For clarity, the transferrin or fusions of transferrin and another protein or proteins can be both a transferrin variant of the invention and have reduced N-linked glycosylation.

It may be particularly advantageous to use a host, for example a yeast, deficient in one or more protein mannosyl transferases involved in O-glycosylation of proteins, for instance by disruption of the gene coding sequence. Recombinantly expressed proteins can be subject to undesirable post-translational modifications by the producing host cell. The mannosylated transferrin would be able to bind to the lectin Concanavalin A. The amount of mannosylated transferrin produced by the yeast can be reduced by using a yeast strain deficient in one or more of the *PMT* genes (WO 94/04687). The most convenient way of achieving this is to create a yeast which has a defect in its genome such that a reduced level of one of the Pmt proteins is produced. For example, there may or may not be a deletion, insertion or transposition in the coding sequence or the regulatory regions (or in another gene regulating the expression of one of the *PMT* genes) such that little or no Pmt protein is produced. Alternatively, the yeast could be transformed to produce an anti-Pmt agent, such as an anti-Pmt antibody. Alternatively, the yeast could be cultured in the presence of a compound that inhibits the activity of one of the PMT genes (Duffy et al, "Inhibition of protein mannosyltransferase 1 (PMT1) activity in the pathogenic yeast Candida albicans", International Conference on Molecular Mechanisms of Fungal Cell Wall Biogenesis, 26-31 August 2001, Monte Verita, Switzerland, Poster Abstract P38; the poster abstract may be viewed at http://www.micro.biol.ethz.ch/cellwall/). If a yeast other than *S. cerevisiae* is used, disruption of one or more of the genes equivalent to the *PMT* genes of S. *cerevisiae* is also beneficial, *e.g.* in *Pichia pastoris* or *Kluyveromyces lactis.* The sequence of *PMT1* (or any other *PMT* gene) isolated from *S. cerevisiae* may be used for the identification or disruption of genes encoding similar enzymatic activities in other fungal species. The cloning of the *PMT1* homologue of *Kluyveromyces lactis* is described in WO 94/04687.

The yeast may or may not also have a deletion of the *HSP150* and/or *YAP3* genes as taught respectively in WO 95/33833 and WO 95/23857.

The transferrin may be produced by recombinant expression and secretion. Where the expression system (i.e. the host cell) is yeast, such as *Saccharomyces cerevisiae,* suitable promoters for *S. cerevisiae* include those associated with the *PGK1* gene, *GAL1* or *GAL10* genes, *TEF1, TEF2, PYK1, PMA1, CYC1, PHO5, TRP1, ADH1, ADH2,* the genes for glyceraldehyde-3-phosphate dehydrogenase, hexokinase, pyruvate decarboxylase, phosphofructokinase, triose phosphate isomerase, phosphoglucose isomerase, glucokinase, $\alpha$-mating factor pheromone, $\alpha$-mating factor pheromone, the *PRB1* promoter, the *PRA1* promoter, the *GPD1* promoter, and hybrid promoters involving hybrids of parts of 5' regulatory regions with parts of 5' regulatory regions of other promoters or with upstream activation sites (e.g. the promoter of EP-A-258 067).

Suitable transcription termination signals are well known in the art. Where the host cell is eukaryotic, the transcription termination signal is preferably derived from the 3' flanking sequence of a eukaryotic gene, which contains proper signals for transcription termination and polyadenylation. Suitable 3' flanking sequences may, for example, be those of the gene naturally linked to the expression control sequence used, i.e. may correspond to the promoter. Alternatively, they may be different. In that case, and where the host is a yeast, preferably *S. cerevisiae,* then the termination signal of the S. *cerevisiae ADH1, ADH2, CYC1,* or *PGK1* genes are preferred.

It may be beneficial for the promoter and open reading frame of the gene encoding the recombinant protein comprising the sequence of a transferrin mutant to be flanked by transcription termination sequences so that the transcription termination sequences are located both upstream and downstream of the promoter and open reading frame, in order to prevent transcriptional read-through into any neighbouring genes, such as 2$\mu$m genes, and vice versa.

In one embodiment, the favoured regulatory sequences in yeast, such as *Saccharomyces cerevisiae,* include: a yeast promoter (e.g. the *Saccharomyces cerevisiae PRB1* promoter), as taught in EP 431 880; and a transcription terminator, preferably the terminator from *Saccharomyces ADH1,* as taught in EP 60 057.

It may be beneficial for the non-coding region to incorporate more than one DNA sequence encoding a translational stop codon, such as UAA, UAG or UGA, in order to minimise translational read-through and thus avoid the production of elongated, non-natural fusion proteins. The translation stop codon UAA is preferred.

It is preferred that the recombinant protein comprising the sequence of a transferrin mutant is secreted. In that case, a sequence encoding a secretion leader sequence may be included in the open reading frame. Thus, a polynucleotide according to the present invention may comprise a sequence that encodes a recombinant protein comprising the sequence of a transferrin mutant operably linked to a polynucleotide sequence that encodes a secretion leader sequence. Leader sequences are usually, although not necessarily, located at the N-terminus of the primary translation product of an ORF and are generally, although not necessarily, cleaved off the protein during the secretion process, to yield the "mature" protein. Thus, in one embodiment, the term "operably linked" in the context of leader sequences includes the meaning that the sequence that encodes a recombinant protein comprising the sequence of a transferrin mutant is linked, at its 5' end, and in-frame, to the 3' end of a polynucleotide sequence that encodes a secretion leader sequence. Alternatively, the polynucleotide sequence that encodes a secretion leader sequence may be located, in-frame, within the coding sequence of the recombinant protein comprising the sequence of a transferrin mutant, or at the 3' end of the coding sequence of the recombinant protein comprising the sequence of a transferrin mutant.

Numerous natural or artificial polypeptide leader sequences (also called secretion pre regions and pre/pro regions) have been used or developed for secreting proteins from host cells. Leader sequences direct a nascent protein towards the machinery of the cell that exports proteins from the cell into the surrounding medium or, in some cases, into the periplasmic space.

For production of proteins in eukaryotic species such as the yeasts *Saccharomyces cerevisiae, Zygosaccharomyces* species, *Kluyveromyces lactis* and *Pichia pastoris,* a secretion leader sequence may be used. This may comprise a signal (pre) sequence or a prepro leader sequence. Signal sequences are known to be heterogeneous in their amino acid sequence (Nothwehr and Gordon 1990, Bioessays 12, 479-484, or Gierasch 1989, Biochemistry 28, p923-930). In essence, signal sequences are generally N-terminally located, have a basic n-region, a hydrophobic h-region and a polar c-region. As long as this structure is retained the signal sequence will work, irrespective of the amino acid composition. How well they work, i.e. how much mature protein is secreted, depends upon the amino acid sequence. Accordingly, the term "signal peptide" is understood to mean a presequence which is predominantly hydrophobic in nature and present as an N-terminal sequence of the precursor form of an extracellular protein expressed in yeast. The function of the signal peptide is to allow the expressed protein to be secreted to enter the endoplasmic reticulum. The signal peptide is normally cleaved off in the course of this process. The signal peptide may be heterologous or homologous to the yeast organism producing the protein. Known leader sequences include those from the S. *cerevisiae* acid phosphatase protein (Pho5p) (see EP 366 400), the invertase protein (Suc2p) (see Smith et al. (1985) Science, 229, 1219-1224) and heat-shock protein-150 (Hsp150p) (see WO 95/33833). Additionally, leader sequences from the S. *cerevisiae* mating factor alpha-1 protein (MFα-1) and from the human lysozyme and human serum albumin (HSA) protein have been used, the latter having been used especially, although not exclusively, for secreting human albumin. WO 90/01063 discloses a fusion of the MFα-1 and HSA leader sequences. In addition, the natural transferrin leader sequence may or may not be used to direct secretion of the recombinant protein comprising the sequence of a transferrin mutant.

The skilled person will appreciate that any suitable plasmid may be used, such as a centromeric plasmid. The examples provide suitable plasmids (centromeric YCplac33-based vectors) for use to transform yeast host cells of the present invention. Alternatively, any other suitable plasmid may be used, such as a yeast-compatible 2μm-based plasmid.

Plasmids obtained from one yeast type can be maintained in other yeast types (Irie et al, 1991, Gene, 108(1), 139-144; Irie et al, 1991, Mol. Gen. Genet., 225(2), 257-265). For example, pSR1 from *Zygosaccharomyces rouxii* can be maintained in *Saccharomyces cerevisiae.* In one embodiment the plasmid may or may not be a 2μm-family plasmid and the host cell will be compatible with the 2μm-family plasmid used (see below for a full description of the following plasmids). For example, where the plasmid is based on pSR1, pSB3 or pSB4 then a suitable yeast cell is *Zygosaccharomyces rouxii;* where the plasmid is based on pSB1 or pSB2 then a suitable yeast cell is *Zygosaccharomyces bailli;* where the plasmid is based on pSM1 then a suitable yeast cell is *Zygosaccharomyces fermentati;* where the plasmid is based on pKD1 then a suitable yeast cell is *Kluyveromyces drosophilarum;* where the plasmid is based on pPM1 then a suitable yeast cell is *Pichia membranaefaciens;* where the plasmid is based on the 2μm plasmid then a suitable yeast cell is *Saccharomyces cerevisiae* or *Saccharomyces carlsbergensis.* Thus, the plasmid may be based on the 2μm plasmid and the yeast cell may be *Saccharomyces cerevisiae.* A 2μm-family plasmid can be said to be "based on" a naturally occurring plasmid if it comprises one, two or preferably three of the genes *FLP, REP1* and REP2 having sequences derived from that naturally occurring plasmid.

Useful yeast episomal plasmid vectors are pRS403-406 and pRS413-416 and are generally available from Stratagene Cloning Systems (La Jolla, CA 92037, USA), YEp24 (Botstein, D., et al. (1979) Gene 8, 17-24), and YEplac122, YEplac195 and YEplac181 (Gietz, R.D. and Sugino. A. (1988) Gene 74, 527-534). Other yeast plasmids are described in WO 90/01063 and EP 424 117, as well as the "disintegration vectors of EP-A-286 424 and WO2005061719. Plasmids pRS403, pRS404, pRS405 and pRS406 are Yeast Integrating plasmids (YIps) and incorporate the yeast selectable markers *HIS3, TRP1, LEU2* and *URA3,* as are YIplac204, YIplac211 and YIplac128 (Gietz, R.D. and Sugino. A. (1988) Gene 74, 527-534). Plasmids pRS413-416 are Yeast Centromere plasmids (YCps) as are YCplac22, YCplac33 and YCplac111 (Gietz, R.D. and Sugino. A. (1988) Gene 74, 527-534).

The processes of the present invention can be used to obtain highly purified transferrin from a relatively impure transferrin solution from a number of sources such as blood products and eukaryotic or prokaryotic cell culture. However, the processes are particularly applicable to purifying recombinant transferrin (rTf), particularly from a yeast such as S. *cerevisiae.* The transferrin produced in accordance with the invention may be any mammalian transferrin such as bovine transferrin, but it is preferably human transferrin or a derivative thereof.

Various references are cited herein, the disclosures of which are incorporated by reference in their entireties.

With reference to the embodiment section of this specification, the invention also provides:

i. A process according to any of embodiments 1 to 27 wherein the final transferrin-containing solution obtained thereby is or is not subsequently subjected to one or more further purification steps.

ii. A process according to any of embodiments 1 to 27 and/or (i), wherein the one or more further purification steps is/are selected from: ammonium sulphate precipitation, ethanol precipitation, acid extraction, solvent extraction, anion exchange chromatography, cation exchange chromatography, phosphocellulose chromatography, hydrophobic interaction chromatography, affinity chromatography, hydroxyapatite chromatography, lectin chromatography, metal affinity/chelating chromatography, concentration, dilution, pH adjustment, diafiltration, ultrafiltration, high performance liquid chromatography ("HPLC"), reverse phase HPLC, conductivity adjustment, removal of matrix-derived dye, removal of yeast-derived colourants and alkaline precipitation of the transferrin to a lipophilic phase.

iii. A process according to any of embodiments 1 to 27 or any of (i) to (ii) wherein the transferrin-containing solution obtained thereby is or is not subsequently further purified by ultrafiltration.

iv. A process according to any of embodiments 1 to 27 or any of (i) to (iii) wherein the transferrin-containing solution obtained thereby is or is not further purified and/or formulated for intravenous administration to a human and/or for use as a cell culture ingredient and/or sterilised and/or placed into a final container.

v. A process according to any of embodiments 1 to 27 or any of (i) to (iv) wherein the transferrin-containing solution obtained thereby is or is not suspended in a liquid suitable for final use of the transferrin.

vi. A process according to any of embodiments 1 to 27 or any of (i) to (v) in which the transferrin is or is not holoized prior to and/or following the anionic chromatographic step and/or the cationic chromatographic step.

vii. A process according to any of embodiments 1 to 27 or any of (i) to (vi) in which the transferrin is or is not apoized prior to and/or following the anionic chromatographic step and/or the cationic chromatographic step.

viii. A process according to any of embodiments 1 to 27 or any of (i) to (vii) in which the relatively impure solution of transferrin is or is not obtained from a microbial cell, such as a fungal cell (preferably a yeast cell such as *Saccharomyces, Pichia or Kluyveromyces)* or a bacterial cell, a mammalian cell or from blood or a blood fraction such as serum.

ix. A process according to any of embodiments 1 to 27 or any of (i) to (viii) in which the transferrin is or is not a naturally occurring transferrin.

x. A process according to any of embodiments 1 to 27 or any of (i) to (ix) in which the transferrin is or is not modified relative to a naturally occurring transferrin.

xi. A process according to (x) which the transferrin comprises or does not comprise SEQ ID No. 4.

xii. A process according to any of embodiments 1 to 27 or any of (i) to (xi) in which the transferrin is or is not a therapeutic transferrin.

xiii. A purified transferrin obtained or obtainable by a process according to any of embodiments 1 to 27 or any of (i) to (xii).

xiv. A cell culture medium comprising a transferrin according to (xiii).

xv. Use of a transferrin according to (xiv) in the manufacture a cell culture medium.

The invention is described, by way of example only, with reference to the drawings and the following, non-limiting, examples:

**EXAMPLES**

**EXAMPLE 1: Cation exchange low pH / high salt wash**

**Recovery data obtained for washing Sepharose Fast Flow (SP-FF) with nine different wash buffers (pH / sodium chloride concentration combinations)**

(a) Low pH/High Salt Wash

Initial Optimization: Twelve runs were performed on an AKTA™ Explorer 100 Air chromatography system (GE Healthcare) on an 8.6 mL column. Washing was carried out using different combinations of pH and salt concentration. Specifically, pH 4.0, 4.5 and 5.1 and sodium chloride concentrations of 0.25, 1.0 and 2.0 M were used. Start material (i.e. solution of about 3.7 mg/ml transferrin) was thawed and conditioned to pH 5.1; 3.5 mS.cm⁻¹ by adding acetic acid. The column was equilibrated at pH 5.0-5.2 and 2.5-3.1 mS.cm⁻¹, conditioned material was loaded at 25 mg transferrin.mL matrix⁻¹ and washed first with buffer of the appropriate pH (i.e. the same pH the wash to be used in wash 2) and no sodium chloride (wash 1). The column was then washed with the low pH/high salt buffer (wash 2) and bound transferrin was eluted with elution buffer (50 mM sodium phosphate, 25 mM sodium chloride, pH 7.0). Load, flow through, wash and eluate fractions were collected, and their transferrin content assayed by GP.HPLC using an area calibration method. This data was used to calculate mass balance and recovery values. Load, wash 2 and eluate fractions were also subjected to yeast antigen (HCP) ELISA analysis, and yeast antigen (HCP) clearance values were calculated.

**Table 2: Affect of wash conditions on recovery of transferrin in eluate**

| Run number | Wash conditions | | Eluate recovery (%) |
|---|---|---|---|
| | NaCl (M) | pH | |
| 1 | 0.25 | 4.0 | 53.9 |
| 2 | 1.0 | 4.0 | 38.7 |
| 3 | 2.0 | 4.0 | 48.4 |
| 4 | 0.25 | 4.5 | 87.2 |
| 5 | 1.0 | 4.5 | 76.3 |
| 6 | 2.0 | 4.5 | 94.0 |
| 7 | 0.25 | 4.5 | 82.1 |
| 8 | 1.0 | 4.5 | 74.1 |
| 9 | 2.0 | 4.5 | 79.7 |
| 10 | 0.25 | 5.1 | 15.2 |
| 11 | 1.0 | 5.1 | 15.9 |
| 12 | 2.0 | 5.1 | 31.7 |

These data (Table 2) show that, of the conditions tested, pH 4.5 at any sodium chloride concentration gives the best recoveries and that at pH 4.5, 2.0 M sodium chloride is the optimum.

**Table 3: Mean recovery and yeast antigen (HCP) clearance data for washing SP-FF with three different pH/ sodium chloride concentration combinations**

| Wash buffer | | Mean eluate recovery (%) | Mean yeast antigen (HCP) clearance (fold) |
|---|---|---|---|
| NaCl (M) | pH | | |
| 0.25 | 4.5 | 84.7 | 24.6 |
| 1 | 4.5 | 75.2 | 27.8 |
| 2 | 4.5 | 86.9 | 25.5 |

Data is a mean of two runs for each sodium chloride concentration. Eluate recovery data is from Table 2. This shows that the wash buffer having 2 M sodium chloride at pH 4.5 gives the best eluate recovery/yeast antigen (HCP) clearance combination.

(b) Further Optimization

This example shows the affect of low pH / high salt wash on yeast antigen (HCP) clearance and transferrin concentration of the eluate.

An SP-FF run was performed on the AKTA Explorer 100 Air on an 8.6 mL column using the pH 4.5, 2 M sodium chloride wash 2 conditions detailed above but with the addition of a third wash step using the equilibration buffer. A further three runs were performed to compare the following three wash regimes:

A: Wash 1 (50 mM sodium acetate pH 5.1)
B: Wash 1 (50 mM sodium acetate pH 4.5); Wash 2 (27 mM sodium acetate, 2 M sodium chloride pH 4.5)
C: Wash 1 (50 mM sodium acetate pH 4.5); Wash 2 (27 mM sodium acetate, 2 M sodium chloride pH 4.5); Wash 3 (50 mM sodium acetate pH 5.1)

Samples from throughout the purifications were subjected to GP.HPLC and ELISA analysis as described above and the data used to calculate transferrin recovery and yeast antigen (HCP) clearance values.

Table 4: Recovery and yeast antigen (HCP) clearance data comparing the three different SP-FF wash regimes

| | SP-FF wash regime | Eluate recovery (%) | Yeast antigen (HCP) clearance (fold) |
|---|---|---|---|
| A | Wash 1: 50 mM sodium acetate pH 5.1 | 72.1 | 16.8 |
| B | Wash 1: 27 mM sodium acetate pH 4.5 <br> Wash 2: 50 mM sodium acetate, 2 M sodium chloride pH 4.5 | 70.9 | 27.6 |
| C | Wash 1: 50 mM sodium acetate pH 4.5 <br> Wash 2: 27 mM sodium acetate, 2 M sodium chloride pH 4.5 <br> Wash 3: 50 mM sodium acetate pH 5.1 | 67.5 | 30.1 |

These data (Table 4) show that the two-wash regime (B) gives a significantly higher yeast antigen (HCP) clearance than the single-wash regime (A) and that the three-wash regime (C) improves on this further with only a relatively small reduction in eluate recovery.

**EXAMPLE 2: Affect of presence of borate in anion exchange chromatography load buffer on the recovery of transferrin from the Q-FF anion exchange chromatography**

Q-FF matrix was obtained from GE Healthcare. Tetraborate was added to SP-FF eluate, from Example 1 (Table 4, wash regime C), at a concentration equivalent to 100 moles borate per mole rTf (equivalent to 0.7 mM borate in the load) and as a control no borate was added. Chromatography was performed as described above using a 0.66cm $\times$ 15cm bed height (5.13 mL) column at a matrix capacity of 30 mg.mL$^{-1}$ matrix using Q-FF. Wash was carried out with 15.7 mM potassium tetraborate. Elution was carried out with 150 mM potassium tetraborate. Fractions were collected during load, wash, elution and NaCl clean and the rTf concentration estimated by RP-HPLC and the recoveries tabulated (Table 5). The flow-through (FT) was also analysed and tabulated (Table 5).

Table 5: Effect of the absence (A) and presence (B) of borate in anion exchange chromatography load buffer on the recovery of transferrin from Q-FF anion exchange chromatography

| A: 0 mol.mol borate in load | | | | B: 100 mol.mol borate in load | | |
|---|---|---|---|---|---|---|
| Fraction | Total (mg) | Recovery (%) | | Fraction | Total (mg) | Recovery (%) |
| Load | 151.7 | | | Load | 150.9 | |
| FT | 43.1 | 28.4 | | FT | 1.7 | 1.1 |
| Wash | 16.4 | 10.8 | | Wash | 19.4 | 12.8 |
| Elution | 75.6 | 49.8 | | Elution | 113.6 | 75.3 |
| NaCl | 12.8 | 29.7 | | NaCl | 13.3 | 8.8 |
| Mass Balance | | 119 | | Mass Balance | | 98 |

Use of borate in the load gave a higher overall recovery of transferrin in the eluate (75.3 % recovery in the presence of borate, 49.8 % recovery in the absence of borate). Also, less transferrin was present in the flow through (FT) when the load included borate (1.1 % in the presence of borate, 28.4 % in the absence of borate).

**EXAMPLE 3: Affect of presence of borate in anion exchange chromatography load buffer on the recovery of transferrin from the Capto™Q anion exchange chromatography**

Capto™Q Matrix was obtained from GE Healthcare. Potassium tetraborate was added to Capto™Q load as follows, SP-FF eluate from Example 1 (Table 4, wash regime C) was diluted 5.7 fold with a 1:1 dilution of 15.7 mM potassium tetraborate in water. Finally, sufficient water and 27% (w/v) NaOH was added to achieve a conductivity of 3.5 mS.cm$^{-1}$ and pH 8.8 (equivalent to 824 mol.mol$^{-1}$, 5.4 mM borate in the load). As a control (no borate) SP-FF eluate was conditioned with water, 27% (w/v) NaOH and 5 M NaCl to achieve pH 8.8 and a conductivity of 3.5 mS.cm$^{-1}$. Sufficient load was applied at flow rate of 0.7 mL.min$^{-1}$ onto a 0.66cm $\times$ 2cm bed height (0.68 mL) column to achieve 40 mg.mL$^{-1}$ matrix. Wash was carried out with 15.7 mM potassium tetraborate. Elution was carried out with 150 mM potassium tetraborate. Fractions were collected during load, wash, elution and NaCl clean and the rTf concentration estimated by RP-HPLC and the recoveries tabulated (Table 6). The flow-through (FT) was also analysed and tabulated (Table 6).

**Table 6: Effect of the absence (A) and presence (B) of borate in anion exchange chromatography load buffer on the recovery of transferrin from Capto™Q anion exchange chromatography**

| A: 0 mol.mol borate in load | | | | B: 825 mol.mol borate in load | | |
|---|---|---|---|---|---|---|
| **Fraction** | **Total (mg)** | **Recovery (%)** | | **Fraction** | **Total (mg)** | **Recovery (%)** |
| **Load** | 28.0 | | | **Load** | 27.0 | |
| **FT** | 8.8 | 31.3 | | **FT** | 3.1 | 11.4 |
| **Wash** | 2.5 | 9.0 | | **Wash** | 1.2 | 4.3 |
| **Elution** | 15.0 | 53.6 | | **Elution** | 21.7 | 80.6 |
| **Mass Balance** | | 94 | | **Mass Balance** | | 96 |

Use of borate in the load gave a higher overall recovery of transferrin in the eluate (80.6 % recovery in the presence of borate, 53.6 % recovery in the absence of borate). Also, less transferrin was present in the flow through (FT) when the load included borate (11.4 % in the presence of borate, 31.3 % in the absence of borate).

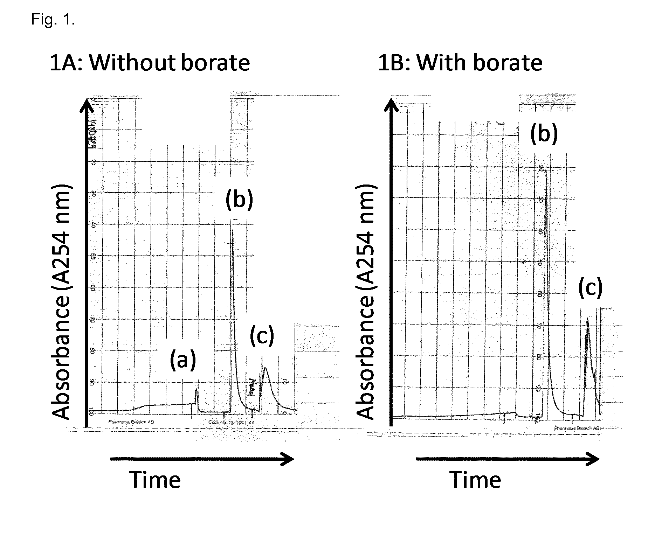

The data are also shown in Figure 1. Figure 1 shows that inclusion of borate in the load improves the binding of transferrin to the column. Figure 1A is a trace for a Capto™Q column for which tetraborate was not added to the transferrin prior to loading onto the column. Figure 1B is a trace for a Capto™Q column for which 824mol.mol$^{-1}$ tetraborate was to the transferrin prior to loading onto the column.

Peak (a) relates to transferrin present in the wash following washing of the column. Peak (b) relates to transferrin present in the eluate. Peak (c) relates to transferrin recovered from the column following washing with NaOH.

The absence of a peak (a) on Figure 1B shows that inclusion of tetraborate in the load improves the binding of transferrin to the column. The presence of peak (a) on Figure 1A shows that loading transferrin on to the column in the absence of tetraborate results in some transferrin being undesirably eluted from the column during the washing step.

**EXAMPLE 4: Low concentration wash of anionic exchange matrix**

Capto™Q chromatography was performed using a 0.66 $\times$ 15cm (5.13 mL) column equilibrated in 15.7 mM potassium tetraborate. SP-FF eluate, from Example 1 (Table 4, wash regime C), was used as load material. The load was conditioned for Capto™Q chromatography by the addition 27% (w/v) NaOH to achieve a load pH of 9.0-9.2 and water to achieve a conductivity -2.6 mS.cm$^{-1}$. Finally, solid potassium tetraborate was added to achieve a final concentration of 5 mM tetraborate (725 mol.mol$^{-1}$) increasing the load conductivity to -3.4 mS.cm$^{-1}$. A column capacity of 30 mg.mL$^{-1}$ matrix was used. Following load, the column was washed with 5 mM potassium tetraborate (10CV), 15.7 mM potassium tetraborate (11 CV) and eluted with 150 mM potassium tetraborate pH 9.4 prior to regeneration of the column with 1 M NaCl, 0.5 M NaOH and 1 hour sanitisation with 1 M NaOH.

**Table 7: Recovery of transferrin over Capto™Q chromatography using SP-FF eluate conditioned with 5 mM tetraborate in the load**

| Fraction | Buffer | Recovery (%) |
|---|---|---|
| Load | pH 9.0, 3.42 mS.cm$^{-1}$ 5 mM potassium tetraborate | 100 |
| FT | - | 1.9 |
| Wash 1 | 5 mM potassium tetraborate | 0.1 |
| Wash 2 | 15.7 mM potassium tetraborate | 0.2 |
| Elution | 150 mM potassium tetraborate | 93.2 |
| Clean 1 | 1 M NaCl + 0.5% (w/v) Polysorbate 80 | 5.5 |
| Mass Balance | | 101.2 |

The data (Table 7) show that the loading of transferrin in the presence of tetraborate, followed by and low concentration wash and a high concentration wash results in low levels of loss of transferrin during each wash step and subsequently a very high level of transferrin (93.2 % recovery) in the eluate.

**EXAMPLE 5: Mathematical modelling of low pH and salt concentration with regards removal of yeast antigen (HCP) and recovery.**

The use of a low pH, high salt concentration wash regime to remove yeast antigens (YA) (i.e. host cell proteins (HCP)) and maintain eluate recovery on SP-FF was investigated further. A Design of Experiments (DoE) approach was employed to allow construction of a mathematical model of the effects of the two factors, pH and salt concentration, on the two responses, eluate recovery and YA clearance. A number of pH and salt concentrations in the range pH 3.66 to 5.34 and 0 mM to 6000 mM sodium chloride were studied. Load material was prepared by adjusting the pH and conductivity of culture supernatant to 5.11 and 3.57mS.cm$^{-1}$ using glacial acetic acid and water. Fifteen Atoll 5mm dia. $\times$ 2.5mm bed height MediaScout® MiniColumns (Atoll GmbH, Weingarten, Germany) containing 50$\mu$L SP-FF matrix (GE Healthcare) were equilibrated with 50mM sodium acetate pH 5.1. This load material was applied at 50$\mu$L.min$^{-1}$ to achieve a 40mg.mL$^{-1}$ matrix loading. Each column was washed first with the appropriate low pH sodium acetate buffer without NaCl (wash 1) and then with a second sodium acetate buffer of equivalent pH but containing the appropriate concentration of NaCl (wash 2) (Table 8). Each column was then eluted with 50mM sodium phosphate, 150mM sodium chloride pH 6.0 and cleaned with 1M NaCl, 0.5% (w/w) polysorbate 80. All chromatographic fractions were collected and the transferrin concentration in each fraction was estimated by reverse phase high performance liquid chromatography (RP.HPLC). YA levels were estimated by enzyme linked immunosorbent assay (ELISA). Recoveries and YA clearance were tabulated and are shown in Table 8.

**Table 8: The effect of varying combinations of low pH and high salt concentration in wash 2 on transferrin recovery and YA clearance in the SP-FF eluate fraction.**

| Run number | Factors | | Responses | |
|---|---|---|---|---|
| | pH | NaCl Concentration (mM) | Recovery (%) | YA clearance (fold) |
| 1 | 4.00 | 5000 | 73.6 | 269 |
| 2 | 4.50 | 2525 | 76.2 | 324 |
| 3 | 3.66 | 2525 | 60.6 | 276 |
| 4 | 4.00 | 50 | 69.7 | 256 |
| 5 | 4.00 | 5000 | 68.0 | 286 |
| 6 | 4.50 | 2525 | 75.6 | 314 |
| 7 | 4.50 | 0 | 81.9 | 298 |
| 8 | 5.34 | 2525 | 26.5 | 213 |

(continued)

| Run number | Factors | | Responses | |
|---|---|---|---|---|
| | pH | NaCl Concentration (mM) | Recovery (%) | YA clearance (fold) |
| 9 | 5.00 | 5000 | 54.9 | 292 |
| 10 | 4.50 | 2525 | 73.6 | 345 |
| 11 | 4.50 | 6000 | 74.4 | 364 |
| 12 | 5.00 | 5000 | 21.9 | 208 |
| 13 | 5.00 | 50 | 81.8 | 356 |
| 14 | 4.00 | 50 | 56.1 | 287 |
| 15 | 5.00 | 50 | 80.8 | 383 |

Data for the recovery and YA clearance at each of the 15 wash conditions ('runs') were entered into the DoE software (Design Expert 6.0.10, Stat-Ease, Inc., Minneapolis, MN, USA) to create a mathematical model of the design space. A computer uses 'design points' (i.e. experimental data points) to generate the mathematical model of the design space. The model was fitted for each of the responses as follows.

(i) Recovery:

- No transformation was required and a quadratic fit was used.
- ANOVA (analysis of variance) data indicated that the fit was significant but that the $salt^2$ term was not required.
- Therefore the $salt^2$ term was removed from the model and the data refitted to produce the following equation:

Recovery =

$$-940.50069 + (441.61018 \times p) + (0.043309 \times s) - (47.57735 \times p^2) - (0.010263 \times p \times s)$$

Where: 'p' = pH and 's' = salt concentration in mM and recovery is % recovery.

(ii) HCP (YA) Clearance:

- No transformation was required and a quadratic fit was used.
- ANOVA data indicated that a quadratic fit was not significant; therefore all terms were included for a cubic fit.
- ANOVA data indicated that the fit was now significant but that the $salt^3$ term was not required.
- Therefore the $salt^3$ term was removed from the model and the data refitted.
- ANOVA data indicated that the fit was significant but that the $salt^2$ term was not required.
- Therefore the $salt^2$ term was removed from the model and the data refitted.
- ANOVA data indicated that the fit was significant but that the $pH \times salt^2$ term was not required as it was aliased with the pH and $pH^3$ terms.
- Therefore the $ph \times salt^2$ term was removed and the data refitted to produce the following equation:

HCP (YA) clearance =

$$16388.65744 - (10512.30681 \times p) - (1.70101 \times s) + (2257.17601 \times p^2) + (0.78628 \times p \times s)$$

$$- (159.06573 \times p^3) - (0.090182 \times p^2 \times s)$$

Where: 'p' = pH and 's' = salt concentration in mM and clearance is 'fold-clearance'.
Using limits of greater than or equal to 60% and greater than or equal to 275-fold ('≥' means 'greater than or equal to'), for acceptable performance of recovery and HCP (YA) clearance respectively, a graphical representation (overlay plot) of a design space was plotted (Figure 2). The areas shaded in grey (*i.e.* I, III and IV) represent those combinations of pH and salt concentration where either recovery and/or HCP (YA) clearance fall outside the acceptable performance limits; whilst the area shown in white (i.e. II) represents those combinations where both pH and salt concentration fall within the acceptable performance limits (i.e. greater than or equal to 60% recovery and greater than or equal to 275-fold HCP (YA) clearance).

### EXAMPLE 6: Addition of potassium tetraborate to Capto Q load

Addition of potassium tetraborate to the Capto Q load material in the range 1 to 1000mol.mol was investigated. Load material was prepared by adjusting the pH and conductivity of SP-FF eluate (from Example 1) to 9.3 and 3.53mS.cm$^{-1}$ using 0.5M NaOH and water. Sufficient 0.5M potassium tetraborate was added to 30mL of this solution to achieve a load sample with a molar ratio of 1000mol borate:1mol transferrin. Individual load samples were prepared by serially diluting the 1000mol.mol solution to 500, 100, 50, 10, 5, 2.5, and 1mol.mol with the pH-adjusted, diluted solution prepared above. The conductivity of each load sample was measured and readjusted to 3.5mS.cm$^{-1}$ with water. Eight Atoll 5mm dia. $\times$ 2.5mm bed height MediaScout® MiniColumns containing 50$\mu$L Capto Q matrix (GE Healthcare) were equilibrated first with 110mM potassium tetraborate and then with 30mM potassium tetraborate. A sufficient volume of each load sample was applied at 50 $\mu$L.min$^{-1}$ to achieve a 40mg.mL$^{-1}$ matrix loading. Each column was washed first with 5mM potassium tetraborate (wash 1) and then with 30mM potassium tetraborate (wash 2) before being eluted with 110mM potassium tetraborate and cleaned with 1M NaCl, 0.5% (w/w) polysorbate 80. All chromatographic fractions were collected and the transferrin concentration estimated by reverse phase high performance liquid chromatography (RP.HPLC). Recoveries were tabulated and are shown in Table 9.

**Table 9: The effect of potassium tetraborate in Capto Q load material between 1 and 1000mol.mol on transferrin recovery in the flow through and eluate fractions.**

| Load conditions | Recovery (%) | | | | | |
|---|---|---|---|---|---|---|
| | Flow through | Wash 1 | Wash 2 | Eluate | Salt clean | Mass Balance |
| 1000mol.mol tetraborate | 0.8 | 0.1 | 1.7 | 93.7 | 5.9 | 102.2 |
| 500mol.mol tetraborate | 2.2 | 0.2 | 2.5 | 91.0 | 5.9 | 101.8 |
| 100mol.mol tetraborate | 3.4 | 0.4 | 3.1 | 78.7 | 5.6 | 91.2 |
| 50mol.mol tetraborate | 6.0 | 0.5 | 2.6 | 75.5 | 4.8 | 89.4 |
| 10mol.mol tetraborate | 17.0 | 0.9 | 1.9 | 62.2 | 4.9 | 87.0 |
| 5mol.mol tetraborate | 12.2 | 1.1 | 1.1 | 48.6 | 4.0 | 87.0 |
| 2.5mol.mol tetraborate | 18.5 | 1.1 | 1.7 | 60.5 | 4.4 | 86.2 |
| 1mol.mol tetraborate | 14.0 | 1.0 | 2.3 | 65.4 | 4.6 | 87.3 |

The data indicated that between 50 and 1000mol.mol the addition of potassium tetraborate to the load was having the desired beneficial effect and reducing the amount of transferrin lost in the flow through fraction, resulting in an increase in the amount recovered in the eluate. Between 10 and 50mol.mol there was a significant increase in the amount of transferrin lost in the flow through resulting in a reduction in the amount recovered in the eluate. In order to better define the cut-off point for the effect of potassium tetraborate addition a second experiment was performed, essentially identical to that described above, but loading at 10, 20, 30, 40 and 50mol.mol. The results of the second experiment are shown in Table 10.

**Table 10: The effect of potassium tetraborate in Capto Q load material between 10 and 50mol.mol on transferrin recovery in the flow through and eluate fractions.**

| Load conditions | Recovery (%) | | | | | |
|---|---|---|---|---|---|---|
| | Flow through | Wash 1 | Wash 2 | Eluate | Salt clean | Mass Balance |
| 50mol.mol tetraborate | 0.5 | 0.1 | 1.2 | 91.1 | 7.9 | 100.7 |
| 40mol.mol tetraborate | 7.3 | 0.4 | 3.6 | 78.8 | 5.7 | 95.8 |
| 30mol.mol tetraborate | 6.9 | 0.5 | 3.1 | 78.7 | 5.9 | 95.0 |
| 20mol.mol tetraborate | 7.5 | 0.5 | 3.4 | 91.0 | 5.8 | 108.2 |
| 10mol.mol tetraborate | 18.8 | 0.9 | 2.1 | 65.7 | 7.2 | 94.7 |

This second set of data indicated that between 20 and 50mol.mol the addition of potassium tetraborate was having the desired beneficial effect and reducing the amount of transferrin lost in the flow through fraction, resulting in an increase in the amount recovered in the eluate. Below 10mol.mol there was a significant increase in the amount of transferrin lost in the flow through resulting in a reduction in the amount recovered in the eluate.

In conclusion the addition of potassium tetraborate to the Capto Q load in the range 20 to 1000mol.mol has a beneficial effect by reducing transferrin losses in the flow through with a resultant increase in the amount of transferrin recovered in the eluate.

**EXAMPLE 7: Addition of potassium tetraborate to Capto Q wash 1**

Addition of potassium tetraborate to the Capto Q wash 1 in the range 0 to 30mM was investigated. Load material was prepared by adjusting the pH and conductivity of SP-FF eluate (from Example 1) to 9.3 and 3.53mS.cm$^{-1}$ using 0.5M NaOH and water. Sufficient 0.5M potassium tetraborate was added to 50mL of this solution to achieve a load sample with a molar ratio of 270mol borate:1 mol transferrin. The conductivity of the load sample was measured and readjusted to 3.5mS.cm$^{-1}$ with water. Nine Atoll 5mm dia. $\times$ 2.5mm bed height MediaScout® MiniColumns containing 50$\mu$L Capto Q matrix (GE Healthcare) were equilibrated first with 110mM potassium tetraborate then with 30mM potassium tetraborate. A sufficient volume of each load sample was applied at 50 $\mu$L.min$^{-1}$ to achieve a 40mg.mL$^{-1}$ matrix loading. Each column was washed first with potassium tetraborate at either 0, 0.1, 0.5, 1, 2, 5, 10, 20 or 30mM (wash 1) and then with 30mM potassium tetraborate (wash 2) before being eluted with 110mM potassium tetraborate and cleaned with 1M NaCl, 0.5% (w/w) polysorbate 80. All chromatographic fractions were collected and the transferrin concentration estimated by RP.HPLC. Recoveries were tabulated and are shown in Table 11.

**Table 11: The effect of potassium tetraborate in Capto Q wash 1 between 0 and 30mM on transferrin recovery in the wash 1 and wash 2 fractions.**

| Wash 1 conditions | Recovery (%) | | | | | |
|---|---|---|---|---|---|---|
| | Flow through | Wash 1 | Wash 2 | Eluate | Salt clean | Mass Balance |
| 30mM tetraborate | 4.5 | 9.5 | 1.2 | 76.1 | 5.3 | 96.6 |
| 20mM tetraborate | 4.0 | 3.1 | 0.9 | 79.5 | 7.4 | 94.9 |
| 10mM tetraborate | 2.6 | 0.3 | 1.5 | 83.8 | 7.2 | 95.4 |
| 5mM tetraborate | 3.4 | 0.1 | 2.2 | 79.5 | 10.9 | 96.2 |

(continued)

| Wash 1 conditions | Recovery (%) | | | | | |
|---|---|---|---|---|---|---|
| | Flow through | Wash 1 | Wash 2 | Eluate | Salt clean | Mass Balance |
| 2mM tetraborate | 5.6 | 0.4 | 2.7 | 80.4 | 6.2 | 95.3 |
| 1 mM tetraborate | 5.6 | 0.5 | 3.5 | 81.0 | 5.1 | 95.7 |
| 0.5mM tetraborate | 6.5 | 0.7 | 4.2 | 80.3 | 5.0 | 96.7 |
| 0.1mM tetraborate | 6.4 | 0.4 | 6.2 | 87.5 | 5.9 | 106.4 |
| 0mM tetraborate | 5.1 | 0.1 | 7.2 | 75.9 | 7.6 | 96.0 |

The data indicated that a concentration of between 0.5 and 20mM potassium tetraborate in wash 1 was having the desired beneficial effect i.e. reducing the amount of transferrin lost in wash 2. Above 20mM there was a significant increase in the amount of transferrin lost in wash 1 whilst between 0 and 0.1 mM there was an unacceptable increase in the amount of transferrin lost in wash 2.

### EXAMPLE 8: Addition of sodium tetraborate to Capto Q load

Addition of sodium tetraborate to the Capto Q load material was investigated. Load material was prepared by adjusting the pH and conductivity of SP-FF eluate to 9.4 and 3.66mS.cm$^{-1}$ using 0.5M NaOH and water. Sufficient 110mM sodium tetraborate was added to half of this solution to achieve a load sample with a molar ratio of 270mol borate:1 mol transferrin. The conductivity of the load sample was measured and readjusted to 3.61 mS.cm$^{-1}$ with water. Two Atoll 5mm dia. $\times$ 2.5mm bed height MediaScout® MiniColumns containing 50$\mu$L Capto Q matrix (GE Healthcare) were equilibrated first with 110mM sodium tetraborate then with 30mM sodium tetraborate. Sufficient of the 270mol.mol load sample and a control of pH adjusted, diluted material containing no sodium tetraborate were applied at 50$\mu$L.min$^{-1}$ to achieve a 40mg.mL$^{-1}$ matrix loading. Each column was washed first with 5mM sodium tetraborate (wash 1) and then with 30mM sodium tetraborate (wash 2) before being eluted with 110mM sodium tetraborate and cleaned with 1M NaCl, 0.5% (w/w) polysorbate 80. All chromatographic fractions were collected and the transferrin concentration estimated by reverse phase high performance liquid chromatography (RP.HPLC). Recoveries were tabulated and are shown in Table 12.

**Table 12: The effect of sodium tetraborate in Capto Q load at 270mol.mol on transferrin recovery in the flow through and eluate fractions.**

| Load conditions | Recovery (%) | | | | | |
|---|---|---|---|---|---|---|
| | Flow through | Wash 1 | Wash 2 | Eluate | Salt clean | Mass Balance |
| 0mol.mol tetraborate | 16.9 | 0.9 | 3.2 | 63.2 | 5.2 | 89.5 |
| 270mol.mol tetraborate | 5.1 | 0.2 | 2.7 | 75.2 | 4.9 | 88.1 |

The data indicated that the addition of sodium tetraborate to the load at 270mol.mol was having the desired beneficial effect i.e. reducing the amount of transferrin lost in the flow through fraction, resulting in an increase in the amount recovered in the eluate.

### EMBODIMENTS

1) A process for purifying a transferrin solution, the process comprising:

a) adding tetraborate to a relatively impure solution of transferrin to provide a solution of transferrin and tetraborate;

b) applying the solution of transferrin and tetraborate to an anionic chromatographic material for which the transferrin has no specific binding activity such that the transferrin binds to the material;

c) washing the chromatographic material to which the transferrin is bound;

d) optionally, further washing the chromatographic material to which the transferrin is bound; and

e) optionally, recovering the transferrin from the anionic chromatographic material.

2) A process according to embodiment 1 wherein: in step (a), the solution of transferrin and tetraborate is at a ratio of at least about 20 mole tetraborate to about 1 mole transferrin.

3) A process according to embodiment 1 or 2 in which the washing of step (c) is carried out using a washing solution having a lower tetraborate concentration than the washing solution used for the washing of step (d).

4) A process according to any preceding embodiment in which the washing of step (c) is carried out using a tetraborate solution of from about 0.1 mM to about 20 mM.

5) A process according to any preceding embodiment in which the washing of step (d) is carried out using a tetraborate solution of from about 5 mM to about 60 mM.

6) A process according to any preceding embodiment comprising:

a) adding tetraborate to a relatively impure solution of transferrin to provide a solution of transferrin and tetraborate at a ratio of at least 20 mole tetraborate to 1 mole transferrin;

b) applying the solution of transferrin and tetraborate to an anionic chromatographic material for which the transferrin has no specific binding affinity such that the transferrin binds to the material;

c) washing the chromatographic material with a tetraborate solution of from 0.1 mM to about 20 mM;

d) subsequently washing the chromatographic material with a tetraborate solution of at least 30 mM; and

e) optionally, recovering the transferrin from the anionic chromatographic material.

7) A process for purifying a transferrin solution, the process comprising an anionic chromatographic step and a cationic chromatographic step, in which:

the cationic chromatographic step comprises:

a) applying a relatively impure solution of a transferrin to a cationic chromatographic material for which the transferrin has no specific affinity such that the transferrin binds to the material;

b) washing the cationic chromatographic material with a wash buffer having a pH of from about 4 to about 5 and being substantially free of salts other than the salt which provides the buffering effect;

c) subsequently washing the cationic chromatographic material with a wash buffer having a pH from about 4 to about 5 and a salt concentration of from about 50 mM to about 5 M; and

d) recovering the transferrin from the cationic chromatographic material; and

the anionic chromatographic step comprises:

a) adding tetraborate to transferrin obtained from step (d) of the cationic chromatographic step to provide a solution of transferrin and tetraborate;

b) applying the solution of transferrin and tetraborate to an anionic chromatographic material for which the transferrin has no specific activity such that the transferrin binds to the material;

c) washing the chromatographic material to which the transferrin is bound;

d) optionally further washing the chromatographic material to which the transferrin is bound; and

e) optionally, recovering the transferrin from the anionic chromatographic material.

8) A process according to any of embodiments 1 to 7 wherein the tetraborate solution used in the washing of step (c) of the anionic chromatographic step is from about 2.5 mM to about 7.5 mM.

9) A process according to embodiment 8 wherein the tetraborate solution used in the washing of step (c) of the anionic chromatographic step is about 5 mM.

10) A process according to any of embodiments 1 to 9 wherein the tetraborate solution used in the washing of step (d) of the anionic chromatographic step is from about 20 mM to about 40 mM.

11)A process according to embodiment 10 wherein the tetraborate solution used in the washing of step (d) of the anionic chromatographic step is about 30 mM.

12)A process according to any of embodiments 1 to 11 wherein the tetraborate is potassium tetraborate or sodium tetraborate.

13)A process according to any of embodiments 1 to 12 wherein the anionic chromatographic material comprises a functional ligand selected from: a diethylaminoethyl (DEAE) group and a quaternary amine group.

14) A process for purifying a transferrin solution, the process comprising a cationic chromatographic step comprising the steps of:

a) applying a relatively impure solution of a transferrin to a cationic chromatographic material for which the transferrin has no specific affinity such that the transferrin binds to the material;
b) washing the chromatographic material with a wash buffer having a pH of from about 4 to about 5 and having a salt concentration of less than 200 mM;
c) subsequently washing the chromatographic material with a wash buffer which:

i) which lies within a design space defined by both of the following equations:

$$\text{(I) Recovery} = -940.50069 + (441.61018 \times p) + (0.043309 \times s) - (47.57735 \times p^2) - (0.010263 \times p \times s)$$

$$\text{(II) Host cell protein clearance} = 16388.65744 - (10512.30681 \times p) - (1.70101 \times s) + (2257.17601 \times p^2) + (0.78628 \times p \times s) - (159.06573 \times p^3) - (0.090182 \times p^2 \times s)$$

where p = pH, s = salt concentration in mM, Recovery is greater than or equal to 60% and YA clearance is greater than or equal to 275-fold; and
ii) has a pH of from 4 to 5 and a salt concentration of from 50 to 5000 mM; and
iii) has a higher salt concentration that the wash buffer of step (b); and

d) optionally, recovering the transferrin from the cationic chromatographic material.

15)A process according to any of embodiments 7 to 14 in which the pH of the wash buffer of step (b) of the cationic chromatographic step is from about 4.3 to about 4.7.

16)A process according to embodiment 16 in which the pH of the wash buffer of step (b) is about 4.5.

17)A process according to any of embodiments 7 to 16 in which the wash buffer of step (b) of the cationic chromatographic step has a salt concentration of less than 50 mM.

18)A process according to embodiment 17 in which the wash buffer of step (b) does not comprise salt other than the salt which provides the buffering effect.

19)A process according to any of embodiments 7 to 18 in which the wash of step (c) of the cationic chromatographic step:

(i) lies within a design space defined by both of the following equations:

(I) Recovery = -940.50069 + (441.61018×p) + (0.043309×s) - (47.57735×p2) - (0.010263×p×s)

(II) Host cell protein clearance = 16388.65744 - (10512.30681×p) - (1.70101×s) + (2257.17601×p2) + (0.78628×p×s) - (159.06573×p3) - (0.090182×p2×s)

where p = pH, s = salt concentration in mM, Recovery is greater than or equal to 60% and YA clearance is greater than or equal to 275-fold; and
(ii) has a pH of from 4 to 5 and a salt concentration of from 50 to 5000 mM.

20) A process according to any of embodiments 7 to 19 in which the pH of the wash buffer of step (c) of the cationic chromatographic step is from about 4.3 to about 4.7.

21) A process according to embodiment 20 in which the pH of the wash buffer of step (c) is about 4.5.

22) A process according to any of embodiments 7 to 21 in which the wash buffer of step (c) of the cationic chromatographic step has a salt concentration of from about 250 mM to about 2.5 M.

23) A process according to embodiment 22 in which the wash buffer of step (c) has a salt concentration of about 2 M.

24) A process according to any of embodiments 7 to 23 in which the salt of step (b) and/or step (c) of the cationic chromatographic step is NaCl.

25) A process according to any of embodiments 7 to 24 in which the cationic chromatographic material comprises a functional ligand comprising a sulphopropyl group.

26) A process according to any of embodiments 7 to 25 in which the cationic chromatographic step further comprises a third wash prior to recovery of the transferrin.

27) A process according to embodiment 26 in which the third wash is carried out using a wash buffer comprising about 50 mM sodium acetate and at about pH 5.1.

## Claims

1. A process for purifying a transferrin solution, the process comprising a cationic chromatographic step comprising the steps of:

    a) applying a relatively impure solution of a transferrin to a cationic chromatographic material for which the transferrin has no specific affinity such that the transferrin binds to the material;
    b) washing the chromatographic material with a wash buffer having a pH of from about 4 to about 5 and having a salt concentration of less than 200 mM;

c) subsequently washing the chromatographic material with a wash buffer which:

i) lies within a design space defined by both of the following equations:

(1) Recovery $= -940.50069 + (441.61018 \times p) + (0.043309 \times s) - (47.57735 \times p^2) - (0.010263 \times p \times s)$

(2) Host cell protein clearance $= 16388.65744 - (10512.30681 \times p) - (1.70101 \times s) + (2257.17601 \times p^2) + (0.78628 \times p \times s) - (159.06573 \times p^3) - (0.090182 \times p^2 \times s)$

where p = pH, s = salt concentration in mM, Recovery is greater than or equal to 60% and YA clearance is greater than or equal to 275-fold; and
ii) has a pH of from 4 to 5 and a salt concentration of from 50 to 5000 mM; and
iii) has a higher salt concentration that the wash buffer of step (b);
iv) and

d) optionally, recovering the transferrin from the cationic chromatographic material.

2. The process according to claim 1 in which the pH of the wash buffer of step (b) of the cationic chromatographic step is from about 4.3 to about 4.7.

3. The process according to claim 2 in which the pH of the wash buffer of step (b) is about 4.5.

4. The process according to any of the preceding claims in which the wash buffer of step (b) of the cationic chromatographic step has a salt concentration of less than 50 mM.

5. The process according to any of the preceding claims in which the wash buffer of step (b) does not comprise salt other than the salt which provides the buffering effect.

6. The process according to any of the preceding claims in which the wash of step (c) of the cationic chromatographic step:

i) lies within a design space defined by both of the following equations:

(1) Recovery $= -940.50069 + (441.61018 \times p) + (0.043309 \times s) - (47.57735 \times p2) - (0.010263 \times p \times s)$

(2) Host cell protein clearance $= 16388.65744 - (10512.30681 \times p) - (1.70101 \times s) + (2257.17601 \times p2) + (0.78628 \times p \times s) - (159.06573 \times p3) - (0.090182 \times p2 \times s)$

where p = pH, s = salt concentration in mM, Recovery is greater than or equal to 60% and YA clearance is greater than or equal to 275-fold; and
ii) has a pH of from 4 to 5 and a salt concentration of from 50 to 5000 mM.

7. A process according to any of the preceding claims in which the pH of the wash buffer of step (c) of the cationic chromatographic step is from about 4.3 to about 4.7.

8. A process according to claim 7 in which the pH of the wash buffer of step (c) is about 4.5.

9. A process according to any of the preceding claims in which the wash buffer of step (c) of the cationic chromatographic step has a salt concentration of from about 250 mM to about 2.5 M.

10. A process according to claim 9 in which the wash buffer of step (c) has a salt concentration of about 2 M.

11. A process according to any of the preceding claims in which the salt of step (b) and/or step (c) of the cationic chromatographic step is NaCl.

12. A process according to any of the preceding claims in which the cationic chromatographic material comprises a functional ligand comprising a sulphopropyl group.

13. A process according to any of the preceding claims in which the cationic chromatographic step further comprises a third wash prior to recovery of the transferrin.

14. A process according to claim 13 in which the third wash is carried out using a wash buffer comprising about 50 mM sodium acetate and at about pH 5.1.

Fig. 1.

1A: Without borate

1B: With borate

Fig. 2

● Design Points

EUROPEAN SEARCH REPORT

Application Number

EP 13 16 3795

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | WO 00/01407 A (SUOMEN PUNAINEN RISTI VERIPALV [FI]; PARKKINEN JAAKKO [FI]; VON BONSDO) 13 January 2000 (2000-01-13) * page 7 - page 11; claims 5-13; example 1 * | 1-14 | INV. C07K14/79 |
| X | WO 2005/003152 A1 (AKZO NOBEL NV [NL]; ROPP PHILIP ALFRED [US]; MURRAY MICHAEL VAN ALEN []) 13 January 2005 (2005-01-13) * page 15 - page 16; claims 1-18 * | 1-14 | |
| A | BLIRUP-JENSEN SOREN: "Protein standardization I: Protein purification procedure for the purification of human prealbumin, orosomucoid and transferrin as primary protein preparations", CLINICAL CHEMISTRY AND LABORATORY MEDICINE, WALTER DE GRUYTER & CO, BERLIN, NEW YORK, vol. 39, no. 11, 1 January 2001 (2001-01-01), pages 1076-1089, XP008106690, ISSN: 1434-6621 * page 1078 * * abstract * | 1-14 | |
| A | WO 97/49414 A (ALPHA THERAPEUTIC CORP [US]) 31 December 1997 (1997-12-31) * page 7; claims 1-15; examples 1-3 * | 1-14 | TECHNICAL FIELDS SEARCHED (IPC) C07K |
| A | WO 03/086272 A (KAMADA LTD [IL]; BAUER SHABTAI [IL]) 23 October 2003 (2003-10-23) * claims 16-56; example 2 * | 1-14 | |

-/--

The present search report has been drawn up for all claims

| Place of search | Date of completion of the search | Examiner |
|---|---|---|
| The Hague | 10 June 2013 | Schmidt-Yodlee, H |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

Europäisches
Patentamt

European
Patent Office

Office européen
des brevets

**EUROPEAN SEARCH REPORT**

Application Number

EP 13 16 3795

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | DE CREMER K ET AL: "Behaviour of vanadate and vanadium-transferrin complex on different anion-exchange columns. Application to in vivo <48>V-labelled rat serum", JOURNAL OF CHROMATOGRAPHY B: BIOMEDICAL SCIENCES & APPLICATIONS, ELSEVIER, AMSTERDAM, NL, vol. 775, no. 2, 5 August 2002 (2002-08-05), pages 143-152, XP004369505, ISSN: 1570-0232 * page 145 * | 1-14 | |
| A | SU 436 854 A1 (PRUIDZE GN [SU]; BOKUCHAVA MA [SU]) 25 July 1974 (1974-07-25) * column 1, line 26 - column 2, line 17; example 1 * | 1-14 | |
| A | WO 02/080980 A (ISIS INNOVATION [GB]; DAVIS BEN [GB]) 17 October 2002 (2002-10-17) * example 2.7.2 * | 1-14 | TECHNICAL FIELDS SEARCHED (IPC) |

The present search report has been drawn up for all claims

| Place of search | Date of completion of the search | Examiner |
|---|---|---|
| The Hague | 10 June 2013 | Schmidt-Yodlee, H |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

# EP 2 617 733 A1

ANNEX TO THE EUROPEAN SEARCH REPORT
ON EUROPEAN PATENT APPLICATION NO.

EP 13 16 3795

This annex lists the patent family members relating to the patent documents cited in the above-mentioned European search report.
The members are as contained in the European Patent Office EDP file on
The European Patent Office is in no way liable for these particulars which are merely given for the purpose of information.

10-06-2013

| Patent document cited in search report | | | Publication date | Patent family member(s) | | | Publication date |
|---|---|---|---|---|---|---|---|
| WO 0001407 | A | | 13-01-2000 | US | 6251860 | B1 | 26-06-2001 |
| | | | | WO | 0001407 | A1 | 13-01-2000 |
| WO 2005003152 | A1 | | 13-01-2005 | AT | 427954 | T | 15-04-2009 |
| | | | | CA | 2530454 | A1 | 13-01-2005 |
| | | | | EP | 1638990 | A1 | 29-03-2006 |
| | | | | ES | 2323642 | T3 | 23-07-2009 |
| | | | | JP | 2007526225 | A | 13-09-2007 |
| | | | | US | 2006189790 | A1 | 24-08-2006 |
| | | | | US | 2011003976 | A1 | 06-01-2011 |
| | | | | WO | 2005003152 | A1 | 13-01-2005 |
| WO 9749414 | A | | 31-12-1997 | AT | 329602 | T | 15-07-2006 |
| | | | | AU | 729682 | B2 | 08-02-2001 |
| | | | | AU | 3303397 | A | 14-01-1998 |
| | | | | CA | 2258740 | A1 | 31-12-1997 |
| | | | | DE | 69736115 | T2 | 14-12-2006 |
| | | | | EP | 0934072 | A1 | 11-08-1999 |
| | | | | ES | 2264167 | T3 | 16-12-2006 |
| | | | | JP | 2002509521 | A | 26-03-2002 |
| | | | | US | 5744586 | A | 28-04-1998 |
| | | | | WO | 9749414 | A1 | 31-12-1997 |
| WO 03086272 | A | | 23-10-2003 | AU | 2002307776 | A1 | 27-10-2003 |
| | | | | US | 2005153875 | A1 | 14-07-2005 |
| | | | | US | 2008102066 | A1 | 01-05-2008 |
| | | | | WO | 03086272 | A2 | 23-10-2003 |
| SU 436854 | A1 | | 25-07-1974 | NONE | | | |
| WO 02080980 | A | | 17-10-2002 | EP | 1372734 | A1 | 02-01-2004 |
| | | | | JP | 2004525171 | A | 19-08-2004 |
| | | | | US | 2004171524 | A1 | 02-09-2004 |
| | | | | US | 2007253942 | A1 | 01-11-2007 |
| | | | | WO | 02080980 | A1 | 17-10-2002 |

EPO FORM P0459

For more details about this annex : see Official Journal of the European Patent Office, No. 12/82

46

## REFERENCES CITED IN THE DESCRIPTION

**Patent documents cited in the description**

- US 6251860 B
- US 5744586 A
- US 5041537 A
- EP 2008060482 W
- US 5986067 A
- WO 2008152140 A
- US 20030221201 A
- US 20040023334 A
- EP 238023 A
- US 5364770 A
- US 5578463 A
- EP 184438 A
- EP 284603 A
- WO 2000056900 A
- WO 9614413 A
- WO 9404687 A
- WO 9533833 A
- WO 9523857 A
- EP 258067 A
- EP 431880 A
- EP 60057 A
- EP 366400 A
- WO 9001063 A
- EP 424117 A
- EP 286424 A
- WO 2005061719 A

**Non-patent literature cited in the description**

- **VAN CAMPENHOUT et al.** *Free Radic. Res.,* 2003, vol. 37, 1069-1077
- **RIVAT et al.** *Journal of Chromatography,* 1992, vol. 576, 71-77
- Protein liquid chromatography. Elsevier, 2000, 18-21
- **LAMBERT et al.** *Comparative Biochemistry and Physiology, Part B,* 2005, vol. 142, 129-141
- **TESTA.** Proteins of iron metabolism. CRC Press, 2002
- **HARRIS ; AISEN.** Iron carriers and iron proteins, Vol. 5, Physical Bioinorganic Chemistry. VCH, 1991, vol. 5
- **MASON et al.** *Biochemistry,* 1993, vol. 32, 5472
- **MASON et al.** *Biochem. J.,* 1998, vol. 330, 35
- **MASON et al.** *Protein Expr. Purif.,* 1996, vol. 8, 119
- **MASON et al.** *Protein Expr. Purif.,* 1991, vol. 2, 214
- **SHIN et al.** *Proc. Natl. Acad. Sci. USA,* 1995, vol. 92, 2820
- **ALI et al.** *J. Biol. Chem.,* 1999, vol. 274, 24066
- **MASON et al.** *Biochemistry,* 2002, vol. 41, 9448
- Protein Formulation and Delivery. Marcel Dekker Inc, 2000
- Rational Design of Stable Protein Formulations - Theory and Practice. Pharmaceutical Biotechnology. Kluwer Academic/Plenum Publishers, 2002, vol. 13
- **YAZDI ; MURPHY.** *Cancer Research,* 1994, vol. 54, 6387-6394
- **WIDERA et al.** *Pharmaceutical Research,* 2003, vol. 20, 1231-1238
- **LEE et al.** *Arch. Pharm. Res.,* 2005, vol. 28, 722-729
- **SANKER.** *Genetic Eng. News,* 2004, vol. 24, 22-28
- **SCHMIDT.** *Appl. Microbiol. Biotechnol.,* 2004, vol. 65, 363-372
- **MASON et al.** *Protein Expr. Purif.,* 2004, vol. 36, 318-326
- **MASON et al.** *Biochemistry,* 2002, vol. 41, 9448-9454
- **LIM et al.** *Biotechnol. Prog.,* 2004, vol. 20, 1192-1197
- **DYCK et al.** *Trends in Biotechnology,* 2003, vol. 21, 394-399
- **MA et al.** *Nature Reviews Genetics,* 2003, vol. 4, 794-805
- **STEINLEIN ; IKEDA.** *Enzyme Microb. Technol.,* 1993, vol. 15, 193-199
- **NAVALAINEN et al.** *Trends in Biotechnology,* 2005, vol. 23, 468-473
- **LIM et al.** *Biotechnol Prog.,* 2004, vol. 20, 1192-1197
- **MASON et al.** *Protein Expr. Purif.,* 2001, vol. 23, 142-150
- **MASON et al.** *Biochemistry,* 1993, vol. 32, 5472-5479
- **SARGENT et al.** *Biometals,* 2006, vol. 19, 513-519

- **MASON et al.** *Biochem. J.,* 1997, vol. 326, 77-85
- **MASON et al.** *Protein Expr. Purif.,* 1996, vol. 8, 119-125
- **STEINLEIN et al.** *Protein Expr. Purif.,* 1995, vol. 6, 619-624
- **MAYSON et al.** *Biotechnol. Bioeng.,* 2003, vol. 81, 291-298
- **DUFFY et al.** Inhibition of protein mannosyltransferase 1 (PMT1) activity in the pathogenic yeast Candida albicans. *International Conference on Molecular Mechanisms of Fungal Cell Wall Biogenesis,* 26 August 2001
- **NOTHWEHR ; GORDON.** *Bioessays,* 1990, vol. 12, 479-484
- **GIERASCH.** *Biochemistry,* 1989, vol. 28, 923-930
- **SMITH et al.** *Science,* 1985, vol. 229, 1219-1224
- **IRIE et al.** *Gene,* 1991, vol. 108 (1), 139-144
- **IRIE et al.** *Mol. Gen. Genet.,* 1991, vol. 225 (2), 257-265
- **BOTSTEIN, D. et al.** *Gene,* 1979, vol. 8, 17-24
- **GIETZ, R.D. ; SUGINO. A.** *Gene,* 1988, vol. 74, 527-534

SEQUENCE LISTING

<110>  Novozymes Biopharma DK A/S

<120>  Purification Process

<130>  11248.000-EP

<160>  4

<170>  PatentIn version 3.5

<210>  1
<211>  679
<212>  PRT
<213>  Homo sapiens


<220>
<221>  MISC_FEATURE
<222>  (1)..(679)
<223>  Homo sapiens transferrin

<400>  1

```
Val Pro Asp Lys Thr Val Arg Trp Cys Ala Val Ser Glu His Glu Ala
1               5                   10                  15


Thr Lys Cys Gln Ser Phe Arg Asp His Met Lys Ser Val Ile Pro Ser
            20                  25                  30


Asp Gly Pro Ser Val Ala Cys Val Lys Lys Ala Ser Tyr Leu Asp Cys
        35                  40                  45


Ile Arg Ala Ile Ala Ala Asn Glu Ala Asp Ala Val Thr Leu Asp Ala
        50                  55                  60


Gly Leu Val Tyr Asp Ala Tyr Leu Ala Pro Asn Asn Leu Lys Pro Val
65                  70                  75                  80


Val Ala Glu Phe Tyr Gly Ser Lys Glu Asp Pro Gln Thr Phe Tyr Tyr
                85                  90                  95


Ala Val Ala Val Val Lys Lys Asp Ser Gly Phe Gln Met Asn Gln Leu
            100                 105                 110


Arg Gly Lys Lys Ser Cys His Thr Gly Leu Gly Arg Ser Ala Gly Trp
            115                 120                 125


Asn Ile Pro Ile Gly Leu Leu Tyr Cys Asp Leu Pro Glu Pro Arg Lys
        130                 135                 140


Pro Leu Glu Lys Ala Val Ala Asn Phe Phe Ser Gly Ser Cys Ala Pro
145                 150                 155                 160
```

Cys Ala Asp Gly Thr Asp Phe Pro Gln Leu Cys Gln Leu Cys Pro Gly
165 170 175

Cys Gly Cys Ser Thr Leu Asn Gln Tyr Phe Gly Tyr Ser Gly Ala Phe
180 185 190

Lys Cys Leu Lys Asp Gly Ala Gly Asp Val Ala Phe Val Lys His Ser
195 200 205

Thr Ile Phe Glu Asn Leu Ala Asn Lys Ala Asp Arg Asp Gln Tyr Glu
210 215 220

Leu Leu Cys Leu Asp Asn Thr Arg Lys Pro Val Asp Glu Tyr Lys Asp
225 230 235 240

Cys His Leu Ala Gln Val Pro Ser His Thr Val Val Ala Arg Ser Met
245 250 255

Gly Gly Lys Glu Asp Leu Ile Trp Glu Leu Leu Asn Gln Ala Gln Glu
260 265 270

His Phe Gly Lys Asp Lys Ser Lys Glu Phe Gln Leu Phe Ser Ser Pro
275 280 285

His Gly Lys Asp Leu Leu Phe Lys Asp Ser Ala His Gly Phe Leu Lys
290 295 300

Val Pro Pro Arg Met Asp Ala Lys Met Tyr Leu Gly Tyr Glu Tyr Val
305 310 315 320

Thr Ala Ile Arg Asn Leu Arg Glu Gly Thr Cys Pro Glu Ala Pro Thr
325 330 335

Asp Glu Cys Lys Pro Val Lys Trp Cys Ala Leu Ser His His Glu Arg
340 345 350

Leu Lys Cys Asp Glu Trp Ser Val Asn Ser Val Gly Lys Ile Glu Cys
355 360 365

Val Ser Ala Glu Thr Thr Glu Asp Cys Ile Ala Lys Ile Met Asn Gly
370 375 380

Glu Ala Asp Ala Met Ser Leu Asp Gly Gly Phe Val Tyr Ile Ala Gly
385 390 395 400

Lys Cys Gly Leu Val Pro Val Leu Ala Glu Asn Tyr Asn Lys Ser Asp
405 410 415

Asn Cys Glu Asp Thr Pro Glu Ala Gly Tyr Phe Ala Val Ala Val Val

```
                        420                      425                         430


        Lys Lys Ser Ala Ser Asp Leu Thr Trp Asp Asn Leu Lys Gly Lys Lys
                435                 440                 445


        Ser Cys His Thr Ala Val Gly Arg Thr Ala Gly Trp Asn Ile Pro Met
                450                 455                 460


        Gly Leu Leu Tyr Asn Lys Ile Asn His Cys Arg Phe Asp Glu Phe Phe
        465                 470                 475                 480


        Ser Glu Gly Cys Ala Pro Gly Ser Lys Lys Asp Ser Ser Leu Cys Lys
                        485                 490                 495


        Leu Cys Met Gly Ser Gly Leu Asn Leu Cys Glu Pro Asn Asn Lys Glu
                        500                 505                 510


        Gly Tyr Tyr Gly Tyr Thr Gly Ala Phe Arg Cys Leu Val Glu Lys Gly
                        515                 520                 525


        Asp Val Ala Phe Val Lys His Gln Thr Val Pro Gln Asn Thr Gly Gly
                530                 535                 540


        Lys Asn Pro Asp Pro Trp Ala Lys Asn Leu Asn Glu Lys Asp Tyr Glu
        545                 550                 555                 560


        Leu Leu Cys Leu Asp Gly Thr Arg Lys Pro Val Glu Glu Tyr Ala Asn
                        565                 570                 575


        Cys His Leu Ala Arg Ala Pro Asn His Ala Val Val Thr Arg Lys Asp
                        580                 585                 590


        Lys Glu Ala Cys Val His Lys Ile Leu Arg Gln Gln Gln His Leu Phe
                595                 600                 605


        Gly Ser Asn Val Thr Asp Cys Ser Gly Asn Phe Cys Leu Phe Arg Ser
                610                 615                 620


        Glu Thr Lys Asp Leu Leu Phe Arg Asp Asp Thr Val Cys Leu Ala Lys
        625                 630                 635                 640


        Leu His Asp Arg Asn Thr Tyr Glu Lys Tyr Leu Gly Glu Glu Tyr Val
                        645                 650                 655


        Lys Ala Val Gly Asn Leu Arg Lys Cys Ser Thr Ser Ser Leu Leu Glu
                        660                 665                 670


        Ala Cys Thr Phe Arg Arg Pro
                675
```

```
<210>   2
<211>   691
<212>   PRT
<213>   Homo sapiens


<220>
<221>   MISC_FEATURE
<222>   (1)..(691)
<223>   Homo sapiens lactoferrin


<400>   2

Gly Arg Arg Arg Ser Val Gln Trp Cys Ala Val Ser Gln Pro Glu Ala
1               5                   10                  15


Thr Lys Cys Phe Gln Trp Gln Arg Asn Met Arg Lys Val Arg Gly Pro
            20                  25                  30


Pro Val Ser Cys Ile Lys Arg Asp Ser Pro Ile Gln Cys Ile Gln Ala
            35                  40                  45


Ile Ala Glu Asn Arg Ala Asp Ala Val Thr Leu Asp Gly Gly Phe Ile
        50                  55                  60


Tyr Glu Ala Gly Leu Ala Pro Tyr Lys Leu Arg Pro Val Ala Ala Glu
65                  70                  75                  80


Val Tyr Gly Thr Glu Arg Gln Pro Arg Thr His Tyr Tyr Ala Val Ala
                85                  90                  95


Val Val Lys Lys Gly Gly Ser Phe Gln Leu Asn Glu Leu Gln Gly Leu
            100                 105                 110


Lys Ser Cys His Thr Gly Leu Arg Arg Thr Ala Gly Trp Asn Val Pro
            115                 120                 125


Ile Gly Thr Leu Arg Pro Phe Leu Asn Trp Ala Gly Pro Pro Glu Pro
        130                 135                 140


Ile Glu Ala Ala Val Ala Arg Phe Phe Ser Ala Ser Cys Val Pro Gly
145                 150                 155                 160


Ala Asp Lys Gly Gln Phe Pro Asn Leu Cys Arg Leu Cys Ala Gly Thr
                165                 170                 175


Gly Glu Asn Lys Cys Ala Phe Ser Ser Gln Glu Pro Tyr Phe Ser Tyr
            180                 185                 190


Ser Gly Ala Phe Lys Cys Leu Arg Asp Gly Ala Gly Asp Val Ala Phe
            195                 200                 205
```

Ile Arg Glu Ser Thr Val Phe Glu Asp Leu Ser Asp Glu Ala Glu Arg
210             215             220

Asp Glu Tyr Glu Leu Leu Cys Pro Asp Asn Thr Arg Lys Pro Val Asp
225             230             235             240

Lys Phe Lys Asp Cys His Leu Ala Arg Val Pro Ser His Ala Val Val
            245             250             255

Ala Arg Ser Val Asn Gly Lys Glu Asp Ala Ile Trp Asn Leu Leu Arg
            260             265             270

Gln Ala Gln Glu Lys Phe Gly Lys Asp Lys Ser Pro Lys Phe Gln Leu
            275             280             285

Phe Gly Ser Pro Ser Gly Gln Lys Asp Leu Leu Phe Lys Asp Ser Ala
290             295             300

Ile Gly Phe Ser Arg Val Pro Pro Arg Ile Asp Ser Gly Leu Tyr Leu
305             310             315             320

Gly Ser Gly Tyr Phe Thr Ala Ile Gln Asn Leu Arg Lys Ser Glu Glu
            325             330             335

Glu Val Ala Ala Arg Arg Ala Arg Val Val Trp Cys Ala Val Gly Glu
            340             345             350

Gln Glu Leu Arg Lys Cys Asn Gln Trp Ser Gly Leu Ser Glu Gly Ser
            355             360             365

Val Thr Cys Ser Ser Ala Ser Thr Thr Glu Asp Cys Ile Ala Leu Val
370             375             380

Leu Lys Gly Glu Ala Asp Ala Met Ser Leu Asp Gly Gly Tyr Val Tyr
385             390             395             400

Thr Ala Gly Lys Cys Gly Leu Val Pro Val Leu Ala Glu Asn Tyr Lys
            405             410             415

Ser Gln Gln Ser Ser Asp Pro Asp Pro Asn Cys Val Asp Arg Pro Val
            420             425             430

Glu Gly Tyr Leu Ala Val Ala Val Val Arg Arg Ser Asp Thr Ser Leu
            435             440             445

Thr Trp Asn Ser Val Lys Gly Lys Lys Ser Cys His Thr Ala Val Asp
450             455             460

```
Arg Thr Ala Gly Trp Asn Ile Pro Met Gly Leu Leu Phe Asn Gln Ala
465             470         475             480

Gly Ser Cys Lys Phe Asp Glu Tyr Phe Ser Gln Ser Cys Ala Pro Gly
            485             490             495

Ser Asp Pro Arg Ser Asn Leu Cys Ala Leu Cys Ile Gly Asp Glu Gln
        500             505             510

Gly Glu Asn Lys Cys Val Pro Asn Ser Asn Glu Arg Tyr Tyr Gly Tyr
        515             520             525

Thr Gly Ala Phe Arg Cys Leu Ala Glu Asn Ala Gly Asp Val Ala Phe
    530             535             540

Val Lys Asp Val Thr Val Leu Gln Asn Thr Asp Gly Asn Asn Asn Glu
545             550             555             560

Ala Trp Ala Lys Asp Leu Lys Leu Ala Asp Phe Ala Leu Leu Cys Leu
            565             570             575

Asp Gly Lys Arg Lys Pro Val Thr Glu Ala Arg Ser Cys His Leu Ala
            580             585             590

Met Ala Pro Asn His Ala Val Val Ser Arg Met Asp Lys Val Glu Arg
        595             600             605

Leu Lys Gln Val Leu Leu His Gln Gln Ala Lys Phe Gly Arg Asn Gly
    610             615             620

Ala Asp Cys Pro Asp Lys Phe Cys Leu Phe Gln Ser Glu Thr Lys Asn
625             630             635             640

Leu Leu Phe Asn Asp Asn Thr Glu Cys Leu Ala Arg Leu His Gly Lys
            645             650             655

Thr Thr Tyr Glu Lys Tyr Leu Gly Pro Gln Tyr Val Ala Gly Ile Thr
            660             665             670

Asn Leu Lys Lys Cys Ser Thr Ser Pro Leu Leu Glu Ala Cys Glu Phe
            675             680             685

Leu Arg Lys
    690


<210>  3
<211>  738
<212>  PRT
<213>  Homo sapiens
```

33

```
<220>
<221>   MISC_FEATURE
<222>   (1)..(738)
<223>   Homo sapiens lactoferrin

<400>   3

Met Arg Gly Pro Ser Gly Ala Leu Trp Leu Leu Leu Ala Leu Arg Thr
1               5                   10                  15

Val Leu Gly Gly Met Glu Val Arg Trp Cys Ala Thr Ser Asp Pro Glu
                20                  25                  30

Gln His Lys Cys Gly Asn Met Ser Glu Ala Phe Arg Glu Ala Gly Ile
            35                  40                  45

Gln Pro Ser Leu Leu Cys Val Arg Gly Thr Ser Ala Asp His Cys Val
        50                  55                  60

Gln Leu Ile Ala Ala Gln Glu Ala Asp Ala Ile Thr Leu Asp Gly Gly
65                  70                  75                  80

Ala Ile Tyr Glu Ala Gly Lys Glu His Gly Leu Lys Pro Val Val Gly
                85                  90                  95

Glu Val Tyr Asp Gln Glu Val Gly Thr Ser Tyr Tyr Ala Val Ala Val
                100                 105                 110

Val Arg Arg Ser Ser His Val Thr Ile Asp Thr Leu Lys Gly Val Lys
            115                 120                 125

Ser Cys His Thr Gly Ile Asn Arg Thr Val Gly Trp Asn Val Pro Val
            130                 135                 140

Gly Tyr Leu Val Glu Ser Gly Arg Leu Ser Val Met Gly Cys Asp Val
145                 150                 155                 160

Leu Lys Ala Val Ser Asp Tyr Phe Gly Gly Ser Cys Val Pro Gly Ala
                165                 170                 175

Gly Glu Thr Ser Tyr Ser Glu Ser Leu Cys Arg Leu Cys Arg Gly Asp
                180                 185                 190

Ser Ser Gly Glu Gly Val Cys Asp Lys Ser Pro Leu Glu Arg Tyr Tyr
            195                 200                 205

Asp Tyr Ser Gly Ala Phe Arg Cys Leu Ala Glu Gly Ala Gly Asp Val
        210                 215                 220
```

34

```
Ala Phe Val Lys His Ser Thr Val Leu Glu Asn Thr Asp Gly Lys Thr
225             230             235             240

Leu Pro Ser Trp Gly Gln Ala Leu Leu Ser Gln Asp Phe Glu Leu Leu
            245             250             255

Cys Arg Asp Gly Ser Arg Ala Asp Val Thr Glu Trp Arg Gln Cys His
            260             265             270

Leu Ala Arg Val Pro Ala His Ala Val Val Val Arg Ala Asp Thr Asp
            275             280             285

Gly Gly Leu Ile Phe Arg Leu Leu Asn Glu Gly Gln Arg Leu Phe Ser
    290             295             300

His Glu Gly Ser Ser Phe Gln Met Phe Ser Ser Glu Ala Tyr Gly Gln
305             310             315             320

Lys Asp Leu Leu Phe Lys Asp Ser Thr Ser Glu Leu Val Pro Ile Ala
            325             330             335

Thr Gln Thr Tyr Glu Ala Trp Leu Gly His Glu Tyr Leu His Ala Met
            340             345             350

Lys Gly Leu Leu Cys Asp Pro Asn Arg Leu Pro Pro Tyr Leu Arg Trp
            355             360             365

Cys Val Leu Ser Thr Pro Glu Ile Gln Lys Cys Gly Asp Met Ala Val
    370             375             380

Ala Phe Arg Arg Gln Arg Leu Lys Pro Glu Ile Gln Cys Val Ser Ala
385             390             395             400

Lys Ser Pro Gln His Cys Met Glu Arg Ile Gln Ala Glu Gln Val Asp
            405             410             415

Ala Val Thr Leu Ser Gly Glu Asp Ile Tyr Thr Ala Gly Lys Thr Tyr
            420             425             430

Gly Leu Val Pro Ala Ala Gly Glu His Tyr Ala Pro Glu Asp Ser Ser
            435             440             445

Asn Ser Tyr Tyr Val Val Ala Val Val Arg Arg Asp Ser Ser His Ala
    450             455             460

Phe Thr Leu Asp Glu Leu Arg Gly Lys Arg Ser Cys His Ala Gly Phe
465             470             475             480

Gly Ser Pro Ala Gly Trp Asp Val Pro Val Gly Ala Leu Ile Gln Arg
```

|  |  |  | 485 |  |  |  |  | 490 |  |  |  |  | 495 |  |
|---|---|---|---|---|---|---|---|---|---|---|---|---|---|---|

Gly Phe Ile Arg Pro Lys Asp Cys Asp Val Leu Thr Ala Val Ser Glu
500                505                510

Phe Phe Asn Ala Ser Cys Val Pro Val Asn Asn Pro Lys Asn Tyr Pro
515                520                525

Ser Ser Leu Cys Ala Leu Cys Val Gly Asp Glu Gln Gly Arg Asn Lys
530                535                540

Cys Val Gly Asn Ser Gln Glu Arg Tyr Tyr Gly Tyr Arg Gly Ala Phe
545                550                555                560

Arg Cys Leu Val Glu Asn Ala Gly Asp Val Ala Phe Val Arg His Thr
565                570                575

Thr Val Phe Asp Asn Thr Asn Gly His Asn Ser Glu Pro Trp Ala Ala
580                585                590

Glu Leu Arg Ser Glu Asp Tyr Glu Leu Leu Cys Pro Asn Gly Ala Arg
595                600                605

Ala Glu Val Ser Gln Phe Ala Ala Cys Asn Leu Ala Gln Ile Pro Pro
610                615                620

His Ala Val Met Val Arg Pro Asp Thr Asn Ile Phe Thr Val Tyr Gly
625                630                635                640

Leu Leu Asp Lys Ala Gln Asp Leu Phe Gly Asp Asp His Asn Lys Asn
645                650                655

Gly Phe Lys Met Phe Asp Ser Ser Asn Tyr His Gly Gln Asp Leu Leu
660                665                670

Phe Lys Asp Ala Thr Val Arg Ala Val Pro Val Gly Glu Lys Thr Thr
675                680                685

Tyr Arg Gly Trp Leu Gly Leu Asp Tyr Val Ala Ala Leu Glu Gly Met
690                695                700

Ser Ser Gln Gln Cys Ser Gly Ala Ala Ala Pro Ala Pro Gly Ala Pro
705                710                715                720

Leu Leu Pro Leu Leu Leu Pro Ala Leu Ala Ala Arg Leu Leu Pro Pro
725                730                735

Ala Leu

```
<210>   4
<211>   679
<212>   PRT
<213>   Artificial sequence

<220>
<223>   Artificial variant of Homo sapiens transferrin

<400>   4
```

Val Pro Asp Lys Thr Val Arg Trp Cys Ala Val Ser Glu His Glu Ala
1               5                   10                  15

Thr Lys Cys Gln Ser Phe Arg Asp His Met Lys Ser Val Ile Pro Ser
            20                  25                  30

Asp Gly Pro Ser Val Ala Cys Val Lys Lys Ala Ser Tyr Leu Asp Cys
        35                  40                  45

Ile Arg Ala Ile Ala Ala Asn Glu Ala Asp Ala Val Thr Leu Asp Ala
    50                  55                  60

Gly Leu Val Tyr Asp Ala Tyr Leu Ala Pro Asn Asn Leu Lys Pro Val
65                  70                  75                  80

Val Ala Glu Phe Tyr Gly Ser Lys Glu Asp Pro Gln Thr Phe Tyr Tyr
                85                  90                  95

Ala Val Ala Val Val Lys Lys Asp Ser Gly Phe Gln Met Asn Gln Leu
            100                 105                 110

Arg Gly Lys Lys Ser Cys His Thr Gly Leu Gly Arg Ser Ala Gly Trp
        115                 120                 125

Asn Ile Pro Ile Gly Leu Leu Tyr Cys Asp Leu Pro Glu Pro Arg Lys
        130                 135                 140

Pro Leu Glu Lys Ala Val Ala Asn Phe Phe Ser Gly Ser Cys Ala Pro
145                 150                 155                 160

Cys Ala Asp Gly Thr Asp Phe Pro Gln Leu Cys Gln Leu Cys Pro Gly
                165                 170                 175

Cys Gly Cys Ser Thr Leu Asn Gln Tyr Phe Gly Tyr Ser Gly Ala Phe
            180                 185                 190

Lys Cys Leu Lys Asp Gly Ala Gly Asp Val Ala Phe Val Lys His Ser
        195                 200                 205

Thr Ile Phe Glu Asn Leu Ala Asn Lys Ala Asp Arg Asp Gln Tyr Glu

37

        210                    215                      220

Leu Leu Cys Leu Asp Asn Thr Arg Lys Pro Val Asp Glu Tyr Lys Asp
225               230               235               240

Cys His Leu Ala Gln Val Pro Ser His Thr Val Val Ala Arg Ser Met
        245               250               255

Gly Gly Lys Glu Asp Leu Ile Trp Glu Leu Leu Asn Gln Ala Gln Glu
        260               265               270

His Phe Gly Lys Asp Lys Ser Lys Glu Phe Gln Leu Phe Ser Ser Pro
        275               280               285

His Gly Lys Asp Leu Leu Phe Lys Asp Ser Ala His Gly Phe Leu Lys
        290               295               300

Val Pro Pro Arg Met Asp Ala Lys Met Tyr Leu Gly Tyr Glu Tyr Val
305               310               315               320

Thr Ala Ile Arg Asn Leu Arg Glu Gly Thr Cys Pro Glu Ala Pro Thr
        325               330               335

Asp Glu Cys Lys Pro Val Lys Trp Cys Ala Leu Ser His His Glu Arg
        340               345               350

Leu Lys Cys Asp Glu Trp Ser Val Asn Ser Val Gly Lys Ile Glu Cys
        355               360               365

Val Ser Ala Glu Thr Thr Glu Asp Cys Ile Ala Lys Ile Met Asn Gly
370               375               380

Glu Ala Asp Ala Met Ser Leu Asp Gly Gly Phe Val Tyr Ile Ala Gly
385               390               395               400

Lys Cys Gly Leu Val Pro Val Leu Ala Glu Asn Tyr Asn Lys Ala Asp
        405               410               415

Asn Cys Glu Asp Thr Pro Glu Ala Gly Tyr Phe Ala Val Ala Val Val
        420               425               430

Lys Lys Ser Ala Ser Asp Leu Thr Trp Asp Asn Leu Lys Gly Lys Lys
        435               440               445

Ser Cys His Thr Ala Val Gly Arg Thr Ala Gly Trp Asn Ile Pro Met
        450               455               460

Gly Leu Leu Tyr Asn Lys Ile Asn His Cys Arg Phe Asp Glu Phe Phe
465               470               475               480

```
Ser Glu Gly Cys Ala Pro Gly Ser Lys Lys Asp Ser Ser Leu Cys Lys
            485             490             495

Leu Cys Met Gly Ser Gly Leu Asn Leu Cys Glu Pro Asn Asn Lys Glu
            500             505             510

Gly Tyr Tyr Gly Tyr Thr Gly Ala Phe Arg Cys Leu Val Glu Lys Gly
            515             520             525

Asp Val Ala Phe Val Lys His Gln Thr Val Pro Gln Asn Thr Gly Gly
    530             535             540

Lys Asn Pro Asp Pro Trp Ala Lys Asn Leu Asn Glu Lys Asp Tyr Glu
545             550             555             560

Leu Leu Cys Leu Asp Gly Thr Arg Lys Pro Val Glu Glu Tyr Ala Asn
            565             570             575

Cys His Leu Ala Arg Ala Pro Asn His Ala Val Val Thr Arg Lys Asp
            580             585             590

Lys Glu Ala Cys Val His Lys Ile Leu Arg Gln Gln Gln His Leu Phe
            595             600             605

Gly Ser Asn Val Ala Asp Cys Ser Gly Asn Phe Cys Leu Phe Arg Ser
    610             615             620

Glu Thr Lys Asp Leu Leu Phe Arg Asp Asp Thr Val Cys Leu Ala Lys
625             630             635             640

Leu His Asp Arg Asn Thr Tyr Glu Lys Tyr Leu Gly Glu Glu Tyr Val
            645             650             655

Lys Ala Val Gly Asn Leu Arg Lys Cys Ser Thr Ser Ser Leu Leu Glu
            660             665             670

Ala Cys Thr Phe Arg Arg Pro
            675
```